# EUROPEAN PATENT SPECIFICATION

(11) **EP 3 107 598 B1**
(45) Date of publication and mention of the grant of the patent: **11.08.2021**
(21) Application number: 15751391.2
(22) Date of filing: 18.02.2015
(51) Int. Cl.: B01J 20/06, B01J 47/024, B01J 20/02, A61M 1/16, B01J 39/08, B01J 20/34, B01J 20/28, B01J 20/26, B01J 20/20, B01J 20/08, B01J 20/30

(54) **PARALLEL MODULES FOR IN-LINE RECHARGING OF SORBENTS USING ALTERNATE DUTY CYCLES**
PARALLELE MODULE ZUM IN-LINE-WIEDERAUFLADEN VON SORBENTIEN MIT ABWECHSELNDEM TASTVERHÄLTNIS
MODULES PARALLELES POUR LA RECHARGE EN CONTINU DE SORBANTS AU MOYEN DE CYCLES DE SERVICE ALTERNES

(30) Priority: 19.02.2014 US 201461941672 P; 26.02.2014 US 201461945073 P; 23.04.2014 US 201414259589
(43) Date of publication of application: 28.12.2016
(73) Proprietor: Medtronic, Inc., Minneapolis, Minnesota 55432 (US)
(72) Inventor: PUDIL, Bryant, J., Plymouth, MN 55447 (US); GERBER, Martin, T., Maple Grove, MN 55369 (US); LURA, David, B., Maple Grove, MN 55311 (US); MEYER, Thomas, E., Stillwater, MN 55082 (US)
(74) Representative: Dehns
(86) International application number: PCT/US2015/016270
(87) International publication number: WO 2015/126877

(56) References cited:
- WO-A2-2013/028809
- US-A1- 2005 274 658
- US-A1- 2006 241 543
- US-A1- 2008 217 245
- US-A1- 2009 101 552
- US-A1- 2009 120 864
- US-A1- 2011 297 593
- None

## Description

### FIELD OF THE INVENTION

The invention relates to parallel modules for in-line recharging sorbents using alternate duty cycles for a sorbent cartridge

### BACKGROUND

Dialysis involves the movement of blood through a dialyzer that has a semi-permeable membrane. Simultaneously, dialysate is circulated through the dialyzer on an opposite side of the semi-permeable membrane. Toxins present in the blood stream of the patient pass from the blood through the membrane into the dialysate. After passing through the dialyzer, the spent dialysate is discarded. Disposal of spent dialysate requires a large amount of source water for preparing the replacement dialysate necessary for use during continuous dialysis. However, in sorbent dialysis systems, the spent dialysate is re-circulated through a sorbent cartridge rather than being discarded. The sorbent cartridge contains layers of sorbent material which selectively remove specific toxins, or break down toxins, in the dialysate.

The advantage of sorbent dialysis is that a much lower amount of water is required. In four hours of traditional dialysis, up to 120 L of water may be required to generate the dialysate. By contrast, using sorbent dialysis, as little as 6 or 7 L of water may be necessary. Thus, the need for drains and a continuous source of purified water are eliminated, rendering the system portable.

One of the drawbacks of sorbent dialysis systems is the high cost. The materials used in the sorbent cartridges can be expensive. Disposing of the cartridges after each use generates waste and drives up costs. Other known dialysate fluid circulation systems and apparatuses have separate housings where a first housing has a material capable of releasing sodium into dialysate fluid flowing through the first housing, and a second housing has a material capable of binding sodium ions from dialysate fluid flowing through the second housing. However, such systems cannot be formed into a single housing design, oftentimes require many liters of water, and may not be portable. The systems also do not provide for recharging some or all of the components of a sorbent cartridge that would allow reuse of specific components and enable lower long-term costs for operating such systems.

Hence, there is a need for a sorbent cartridge having separation of materials within the sorbent cartridge into modules to allow for isolation of those materials. There is a need for a sorbent cartridge providing for isolation of one or more sorbent materials to allow for cheaper or non-reusable materials to be discarded, while more expensive and reusable materials are recharged. There is a further need for a unitary sorbent cartridge having multiple discreet modules that can be easily connected and/or detached from the unitary sorbent cartridge thereby facilitating the recharging and/or recycling of the sorbent materials and the sorbent cartridge while retaining a single unitary design. There is also a need for a sorbent cartridge having the features of reduced size and weight necessary for a portable dialysis machine. There is a need for a modular sorbent cartridge wherein the sorbent materials can be arranged within the modules of the cartridge to allow for isolation of particular materials or groups of materials. There is a further need for any one of the modules in the cartridge to be reusable or optionally detachable from the cartridge to allow any one of disposal, recycling or recharging of sorbent material within the module. There is a need for a sorbent cartridge having specific materials that can be recharged and allowing for disposal of less expensive materials.

There is a need for the sorbent materials to be recharged without removing the modules from the sorbent cartridge during operation, making the system easier to use. There is a need for a recharging means directly attached to the sorbent modules, to allow the modules to be recharged simply by directing fluid flow from the rechargers to the module. There is further a need for one or more of the modules to be removable to allow for the recycling and/or disposal of these modules, while allowing the recharging of other modules. There is a need for recharging the sorbent modules in an alternate duty cycle to recharge the material without disrupting the other modules.

Sorbent cartridges and dialysis systems are taught in US 2006/241543, US 2008/217245 and US 2009/101552.

### SUMMARY OF THE INVENTION

The first aspect of the invention provides a sorbent cartridge as claimed in claim 1.

In any embodiment of the first aspect of the invention, the modular sorbent cartridge can have valves positioned before and/or after the modules on the connectors to selectively direct flow through the modules, fluid flow paths, wash lines, or bypass lines.

In any embodiment of the first aspect of the invention, the modular sorbent cartridge can have valves positioned before and/or after the modules on the connectors to selectively direct flow through the modules.

In any embodiment of the first aspect of the invention, the valve can be any one or two-way, three-way, four-way valves or combinations thereof.

In any embodiment of the first aspect of the invention, at least one of the modules is configured to be in an offline state by being fluidly connectable to one or more recharger, and at least one of the modules is configured to be in an online state by being fluidly connectable to any one of the fluid flow path or the bypass line.

In any embodiment of the first aspect of the invention, a first module can be positioned in series before a second and third module, wherein the second and third module can be positioned parallel to one another.

In any embodiment of the first aspect of the invention, the first module can be connected to a first connector; the first connector having a first valve, wherein the first valve can connect the first connector, a second connector, a third connector, and a fourth connector. The second connector can connect the first valve to the second module. The third connector can connect the first valve to the third module. The fourth connector can connect the first valve to a second valve, wherein the second valve can connect the fourth connector, a fifth connector, a sixth connector, and a seventh connector. The fifth connector can connect the second valve to the second module. The sixth connector can connect the second valve to the third module. The seventh connector can connect the second valve to another section of the sorbent cartridge.

In any embodiment of the first aspect of the invention, a first module can be positioned in series before a second and third module, wherein the second and third module can be positioned parallel to one another. The first module can be connected to a first connector; the first connector having a first valve.

In any embodiment of the first aspect of the invention, the first valve can connect the first connector, a second connector, a third connector, and a fourth connector. The second connector can connect the first valve to a second valve, wherein the second valve can connect the second connector, a first wash line and the second module. The first wash line can connect the second valve to a first recharger connector. The third connector can connect the first valve to a third valve, wherein the third valve can connect the third connector, a second wash line and the third module. The second wash line can connect the third valve to a second recharger connector. The fourth connector can connect the first valve to a sixth valve, wherein the sixth valve can connect the fourth connector, a fifth connector, a sixth connector, and a seventh connector. The fifth connector can connect the sixth valve to a fourth valve, wherein the fourth valve can connect the fifth connector, a third wash line and the second module. The third wash line can connect the fourth valve to a third recharger connector. The sixth connector can connect the sixth valve to a fifth valve, wherein the fifth valve can connect the sixth connector, a fourth wash line and the third module. The fourth wash line can connect the fifth valve to a fourth recharger connector. The seventh connector can connect the sixth valve to another section of the sorbent cartridge.

In any embodiment of the first aspect of the invention, a first and second module can be positioned parallel to one another, a third and fourth module can be positioned parallel to one another, and the first and second modules can be in series with the third and fourth modules, respectively.

In any embodiment of the first aspect of the invention, a first valve can connect a first, second, third and fourth connector. The second connector can connect the first valve to a second valve. The second valve can connect the second connector, a first wash line and the first module. The first wash line can connect the second valve to a first recharger connector. The third connector can connect the first valve to a third valve. The third valve can connect the third connector, a second wash line, and the second module. The second wash line can connect the third valve to a second recharger connector. The fourth connector can connect the first valve to a tenth valve. The tenth valve can connect the fourth connector, a fifth connector, a sixth connector and an eleventh valve. The fifth connector can connect the tenth valve to a fourth valve. The forth valve can connect the fifth connector, a third wash line, and the first module. The third wash line can connect the fourth valve to a third recharger connector. The sixth connector can connect the tenth valve to a fifth valve. The fifth valve can connect the sixth connector, a fourth wash line and the second module. The fourth wash line can connect a fourth recharger connector. The eleventh valve can connect the fourth connector, a seventh connector, an eighth connector and a twelfth valve. The seventh connector can connect the eleventh valve to a sixth valve. The sixth valve can connect the seventh connector, a fifth wash line and the third module. The fifth wash line can connect a fifth recharger connector. The eighth connector can connect the eleventh valve to a seventh valve. The seventh valve can connect the eighth connector, a sixth wash line and the fourth module. The sixth wash line can connect the seventh valve to a sixth recharger connector. The twelfth valve can connect the fourth connector, a ninth connector and a tenth connector. The ninth connector can connect the twelfth valve to an eighth valve. The eighth valve can connect the ninth connector, a seventh wash line and the third module. The seventh wash line can connect the eighth valve to a seventh recharger connector. The tenth connector can connect the twelfth valve to a ninth valve. The ninth valve can connect the tenth connector, an eighth wash line and the fourth module. The eighth wash line can connect the ninth valve to an eighth recharger connector.

In any embodiment of the first aspect of the invention, a first and second module are positioned parallel to one another, a third and fourth module can be positioned parallel to one another, and the first and second modules can be in series with the third and fourth modules, respectively.

In any embodiment of the first aspect of the invention, a first valve can connect a first, second, third and fourth connector. The second connector can connect the first valve to a second valve. The second valve can connect the second connector, a first wash line and the first module. The first wash line can connect the second valve to a first recharger connector. The third connector can connect the first valve to a third valve. The third valve can connect the third connector, a second wash line and the second module. The second wash line can connect the third valve to a second recharger connector. The fourth connector can connect the first valve to a twelfth valve. The twelfth valve can connect the fourth connector, a fifth connector, a sixth connector and a thirteenth valve. The fifth connector can connect the twelfth valve to a sixth valve. The sixth valve can connect the fifth connector, a fourth valve and an eighth valve. The fourth valve can connect the sixth valve, a third wash line and the first module. The third wash line can connect the fourth valve to a third recharger connector. The sixth connector can connect the twelfth valve to a seventh valve. The seventh valve can connect the sixth connector, a ninth valve and a fifth valve. The fifth valve can connect the seventh valve, a fourth wash line and the second module. The fourth wash line can connect the fifth valve to a fourth recharger connector. The eighth valve can connect the sixth valve, a fifth wash line and the third module. The fifth wash line can connect the eighth valve to a fifth recharger connector. The ninth valve can connect the seventh valve, a sixth wash line and the fourth module. The sixth wash line can connect the ninth valve to a sixth recharger connector. The thirteenth valve can connect the fourth connector, a seventh connector and an eighth connector. The seventh connector can connect the thirteenth valve to a tenth valve. The tenth valve can connect the seventh connector, a seventh wash line and the third module. The seventh wash line can connect the tenth valve to a seventh recharger connector. The eighth connector can connect the thirteenth valve to an eleventh valve. The eleventh valve can connect the eighth connector, an eighth wash line and the fourth module. The eighth wash line can connect the eleventh valve to an eighth recharger connector.

In any embodiment of the first aspect of the invention, a first and second module are positioned parallel to one another, a third and fourth module can be positioned parallel to one another, a fifth and sixth module can positioned parallel to one another and the first and second modules can be in series with the third and fourth modules, and the third and fourth modules can be in series with the fifth and sixth modules, respectively.

In any embodiment of the first aspect of the invention, a first valve can connect a first, second, third and fourth connector. The second connector can connect the first valve to a second valve. The second valve can connect the second connector, a first wash line and the first module. The first wash line can connect the second valve to a first recharger connector. The third connector can connect the first valve to a third valve. The third valve can connect the third connector, a second wash line, and the second module. The second wash line can connect the third valve to a second recharger connector. The fourth connector can connect the first valve to a fourteenth valve. The fourteenth valve can connect the fourth connector, a fifth connector, a sixth connector and a fifteenth valve. The fifth connector can connect the fourteenth valve to a fourth valve. The forth valve can connect the fifth connector, a third wash line, and the first module. The third wash line can connect the fourth valve to a third recharger connector. The sixth connector can connect the fourteenth valve to a fifth valve. The fifth valve can connect the sixth connector, a fourth wash line and the second module. The fourth wash line can connect a fourth recharger connector. The fifteenth valve can connect the fourth connector, a seventh connector, an eighth connector and a sixteenth valve. The seventh connector can connect the fifteenth valve to a sixth valve. The sixth valve can connect the seventh connector, a fifth wash line and the third module. The fifth wash line can connect a fifth recharger connector. The eighth connector can connect the fifteenth valve to a seventh valve. The seventh valve can connect the eighth connector, a sixth wash line and the fourth module. The sixth wash line can connect the seventh valve to a sixth recharger connector. The sixteenth valve can connect the fourth connector, a ninth connector, a tenth connector and a seventeenth valve. The ninth connector can connect the sixteenth valve to an eighth valve. The eighth valve can connect the ninth connector, a seventh wash line and the third module. The seventh wash line can connect the eighth valve to a seventh recharger connector. The tenth connector can connect the sixteenth valve to a ninth valve. The ninth valve can connect the tenth connector, an eighth wash line and the fourth module. The eighth wash line can connect the ninth valve to an eighth recharger connector. The seventeenth valve can connect the fourth connector, an eleventh connector, a twelfth connector and an eighteenth valve. The eleventh connector can connect the seventeenth valve to a tenth valve. The tenth valve can connect the eleventh connector, a ninth wash line and the fifth module. The ninth wash line can connect the tenth valve to a ninth recharger connector. The twelfth connector can connect the seventeenth valve to an eleventh valve. The eleventh valve can connect the twelfth connector, a tenth wash line and the sixth module. The tenth wash line can connect the eleventh valve to a tenth recharger connector. The eighteenth valve can connect the fourth connector, a thirteenth connector and a fourteenth connector. The thirteenth connector can connect the eighteenth valve to a twelfth valve. The twelfth valve can connect the thirteenth connector, an eleventh wash line and the fifth module. The eleventh wash line can connect the twelfth valve to an eleventh recharger connector. The fourteenth connector can connect the eighteenth valve to a thirteenth valve. The thirteenth valve can connect the fourteenth connector, a twelfth wash line and the sixth module. The twelfth wash line can connect the thirteenth valve and a twelfth recharger connector.

In any embodiment of the first aspect of the invention, the sorbent cartridge comprises at least one reusable module having one or more connectors.

In any embodiment of the first aspect of the invention, the cartridge can comprise at least one non-reusable module.

In any embodiment of the first aspect of the invention, the at least one reusable module contains sorbent material.

In any embodiment of the first aspect of the invention, the at least one reusable module can contain multiple sorbent materials.

In any embodiment of the first aspect of the invention, the at least one non-reusable module contains sorbent material.

In any embodiment of the first aspect of the invention, the at least one non-reusable module can contain multiple sorbent materials.

In any embodiment of the first aspect of the invention, the connectors may be selected from the group comprising quick-connect, twist lock, push-on or threaded fitting.

In any embodiment of the first aspect of the invention, the connectors may comprise a length of tubing and a valve assembly.

In any embodiment of the first aspect of the invention, the sorbent material in the reusable module may be selected from a group comprising zirconium phosphate, hydrous zirconium oxide, activated carbon, alumina, urease, and ion exchange resin.

In any embodiment of the first aspect of the invention, the ion exchange resin can be selected to only remove calcium and magnesium ions by using a chelating ion exchange resin. The respective layers can be formed into any combination of layers without restriction.

In any embodiment of the first aspect of the invention, the sorbent material in the non-reusable module may be selected from a group comprising zirconium phosphate, hydrous zirconium oxide, activated carbon, alumina, urease, and ion exchange resin.

In any embodiment of the first aspect of the invention, the ion exchange resin can be selected to only remove calcium and magnesium ions by using a chelating ion exchange resin. The respective layers can be formed into any combination of layers without restriction.

In any embodiment of the first aspect of the invention, the reusable module can be detachable from the sorbent cartridge.

In any embodiment of the first aspect of the invention, the reusable module can be recyclable.

In any embodiment of the first aspect of the invention, the modules may have a barcode or other identification system.

In any embodiment of the first aspect of the invention, the connectors may have an access point for a sensor.

In any embodiment of the first aspect of the invention, the at least two modules are part of a controlled compliant dialysis circuit.

In any embodiment of the first aspect of the invention, the valves can be operated under control of a programmable controller or computer system to regulate flow into, out of, and between modules.

In any embodiment of the first aspect of the invention, the fluid flow through the valves can be sensed by a photocell or other flow sensing and/or measuring apparatus.

In any embodiment of the first aspect of the invention, a control pump can be utilized to circulate fluid in the fluid flow path.

In any embodiment of the first aspect of the invention, the multiple sorbent materials may be mixed together.

In any embodiment of the first aspect of the invention, the sorbent cartridge has a first and second module positioned parallel to one another, can have a third and fourth module positioned parallel to one another, and a fifth and sixth module positioned parallel to one another. The first and second modules can be in series with the third and fourth modules, and the third and fourth modules can be in series with the fifth and sixth modules, respectively.

In any embodiment of the first aspect of the invention, the first and second modules can be fluidly connected to a first set of one or more valves positioned on a first set of one or more connectors before the first and second modules, such that fluid may be directed into either the first or second module. A bypass line can be fluidly connected to the first set of one or more valves such that fluid can bypass both the first and second module. A first recharger connector can be fluidly connected to the first set of one or more valves such that fluid may be directed from the first recharger connector to either the first or second module. The first module, second module, and bypass line can be fluidly connected to a second set of one or more valves positioned on a second set of one or more connectors after the first and second modules and before the third and fourth modules, such that fluid may be directed from the first or second module into either the third or fourth module. The bypass line can be fluidly connected to the second set of one or more valves such that fluid can bypass both the third and fourth module. A second recharger connector can be fluidly connected to the second set of one or more valves such that fluid may be directed from the second recharger connector to either the third or fourth module. The third module, fourth module, and bypass line can be fluidly connected to a third set of one or more valves positioned on a third set of one or more connectors after the third and fourth modules and before the fifth and sixth modules, such that fluid may be directed from the third or fourth module into either the fifth or sixth module. The bypass line can be fluidly connected to the third set of one or more valves such that fluid can bypass both the fifth and sixth module. A third recharger connector can be fluidly connected to the third set of one or more valves such that fluid may be directed from the third recharger connector to either the fifth or sixth module.

In any embodiment of the first aspect of the invention, a first and second module are positioned parallel to one another, a third and fourth module can be positioned parallel to one another, and the first and second modules can be in series with the third and fourth modules, respectively.

In any embodiment of the first aspect of the invention, the first and second modules can be fluidly connected to a first set of one or more valves positioned on a first set of one or more connectors before the first and second modules, such that fluid may be directed into either the first or second module. A bypass line can be fluidly connected to the first set of one or more valves such that fluid can bypass both the first and second module. A first recharger connector can be fluidly connected to the first set of one or more valves such that fluid may be directed from the first recharger connector to either the first or second module. The first module, second module, and bypass line can be fluidly connected to a second set of one or more valves positioned on a second set of one or more connectors after the first and second modules and before the third and fourth modules, such that fluid may be directed from the first or second module into either the third or fourth module. The bypass line can be fluidly connected to the second set of one or more valves such that fluid can bypass both the third and fourth module. A second recharger connector can be fluidly connected to the second set of one or more valves such that fluid may be directed from the second recharger connector to either the third or fourth module.

In any embodiment of the first aspect of the invention, a first module can be positioned in series before a second and third module. The second and third module can be positioned parallel to one another. The first module can be connected to a set of one or more connectors positioned after the first module and before the second and third modules. A set of one or more valves can be positioned on the set of one or more connectors such that fluid may be directed into either the second or third module. A bypass line can be fluidly connected to the set of one or more valves such that fluid can bypass both the second and third module. A recharger connector can be fluidly connected to the set of one or more valves such that fluid may be directed from the recharger connector to either the second or third module.

In any embodiment of the first aspect of the invention, a first and second module are positioned parallel to one another, a third and fourth module can be positioned parallel to one another, and a fifth and sixth module can be positioned parallel to one another. The first and second modules can be in series with the third and fourth modules, and the third and fourth modules can be in series with the fifth and sixth modules, respectively.

In any embodiment of the first aspect of the invention, the first and second modules can be fluidly connected to a first set of one or more valves positioned on a first set of one or more connectors before the first and second modules, such that fluid may be directed into either the first or second module. A bypass line can be fluidly connected to the first set of one or more valves such that fluid can bypass both the first and second module. The first module, second module, and bypass line can be fluidly connected to a second set of one or more valves positioned on a second set of one or more connectors after the first and second modules and before the third and fourth modules, such that fluid may be directed from the first or second module into either the third or fourth module. The bypass line can be fluidly connected to the second set of one or more valves such that fluid can bypass both the third and fourth module. The third module, fourth module, and bypass line can be fluidly connected to a third set of one or more valves positioned on a third set of one or more connectors after the third and fourth modules and before the fifth and sixth modules, such that fluid may be directed from the third or fourth module into either the fifth or sixth module. The bypass line can be fluidly connected to the third set of one or more valves such that fluid can bypass both the fifth and sixth module.

In any embodiment of the first aspect of the invention, a first and second module are positioned parallel to one another, a third and fourth module can be positioned parallel to one another, and the first and second modules can be in series with the third and fourth modules, respectively.

In any embodiment of the first aspect of the invention, the first and second modules can be fluidly connected to a first set of one or more valves positioned on a first set of one or more connectors before the first and second modules, such that fluid may be directed into either the first or second module. A bypass line can be fluidly connected to the first set of one or more valves such that fluid can bypass both the first and second module. The first module, second module, and bypass line can be fluidly connected to a second set of one or more valves positioned on a second set of one or more connectors after the first and second modules and before the third and fourth modules, such that fluid may be directed from the first or second module into either the third or fourth module. The bypass line can be fluidly connected to the second set of one or more valves such that fluid can bypass both the third and fourth module.

In any embodiment of the first aspect of the invention, a first module can be positioned in series before a second and third module. The second and third module can be positioned parallel to one another. The first module can be connected to a set of one or more connectors positioned after the first module and before the second and third modules. A set of one or more valves can be positioned on the set of one or more connectors such that fluid may be directed into either the second or third module. A bypass line can be fluidly connected to the set of one or more valves such that fluid can bypass both the second and third module.

Any of the features disclosed as being part of the first aspect of the invention can be included in the first aspect of the invention, either alone or in combination.

Further described but not claimed is a fluid circuit. The fluid circuit can have at least two modules in parallel, each module connected by one or more connectors. The circuit can have an operational line directing flow along the connectors and through the modules. The circuit can have at least one wash line fluidly connecting one or more connectors to a recharger. The fluid circuit can also have at least one bypass line to bypass at least one module and operational line.

The operational line directing flow along the connectors through the modules can be fluidly connected to one or more modules in an online state.

The operational line directing flow along the connectors through the modules can be fluidly connected to one or more modules in an offline state.

Any of the features disclosed above can be included in fluid circuit, either alone or in combination.

Further described but not claimed is a method of recharging a sorbent. This method can comprise connecting at least a first and second module in parallel with one or more connectors. The method can comprise fluidly connecting at least one connector to a wash line, wherein the wash line can be fluidly connected to a recharger. The method can comprise fluidly connecting at least one connector to a bypass line wherein the bypass line can divert flow from the connector to bypass at least one module. The method can comprise connecting one or more valves to the connectors at junctions between the modules, bypass lines and/or wash lines. The method can comprise selectively opening and closing the valves to direct the flow through the connectors, modules, bypass lines and/or wash lines.

The valves can be an either two-way, three-way, or four-way valves, or combinations thereof.

A valve can be positioned on a connector before the first module, the valve connecting the connector, a wash line and the second module. The valve can be open to the wash line and closed to the connector and the second module such that flow is directed to a recharger.

A valve can be positioned on a connector before the first module, the valve connecting the connector, a wash line and the second module. The valve can be open to the wash line and connector and closed to the second module such that flow is directed to a recharger.

A valve can be positioned on a connector before the first module, the valve connecting the connector, a wash line and the second module. The valve can be open to the second module, and closed to the wash line and connector, such that flow is directed to the second module.

A valve can be positioned on a connector before the first module, the valve connecting the connector, a wash line and the second module. The valve can be closed to the second module and wash line, such that flow is directed through the connector and through the first module.

A pump can be attached to the recharger or wash line.

The method can comprise using gas to blow out the module.

A gas, such as argon gas, nitrogen gas and air, can be used to blow out the module.

The wash lines can be subdivided into a top and bottom line.

The top line can be a liquid line and the bottom line can be a gas line.

The top line can be a gas line and the bottom line can be a liquid line.

The top and bottom lines can be liquid lines.

Any of the features disclosed above can be included in the method of recharging the sorbent, either alone or in combination.

### BRIEF DESCRIPTION OF THE DRAWINGS

FIG. 1 shows a sorbent cartridge containing activated carbon, hydrous zirconium oxide, urease, alumina, and zirconium phosphate.
FIG. 2 shows a modular sorbent cartridge with two modules.
FIG. 3 shows a modular sorbent cartridge with two modules including activated carbon, alumina, urease, and zirconium oxide in the first module and zirconium phosphate in the second module.
FIG. 4 shows a method for recharging the zirconium phosphate sorbent material.
FIG. 5 shows a modular sorbent cartridge with two modules including activated carbon, zirconium phosphate, urease, alumina, and hydrous zirconium oxide in the first module and zirconium phosphate in the second module.
FIG. 6 shows a modular sorbent cartridge with two modules including activated carbon, ion exchange resin, alumina, urease, and hydrous zirconium oxide in the first module and zirconium phosphate in the second module.
FIG. 7 shows a modular sorbent cartridge with two modules including activated carbon, alumina, urease, and zirconium phosphate in the first module and hydrous zirconium oxide and zirconium phosphate in the second module.
FIG. 8 shows a modular sorbent cartridge with two modules including activated carbon, alumina, urease, and hydrous zirconium oxide in the first module and ion exchange resin and zirconium phosphate in the second module.
FIG. 9 shows a modular sorbent cartridge with two modules including activated carbon, alumina, and urease in the first module and zirconium oxide, ion exchange resin, and zirconium phosphate in the second module.
FIG. 10 shows a modular sorbent cartridge with three modules including activated carbon, alumina, urease, and hydrous zirconium oxide in the first module, zirconium phosphate in the second module, and zirconium phosphate and activated carbon in the third module.
FIG. 11 shows a modular sorbent cartridge with three modules including activated carbon in the first module, alumina and urease in the second module, and ion-exchange resin, zirconium phosphate, and hydrous zirconium oxide in the third module, with an optional bypass line to direct fluid from the first to the third modules.
FIG. 12 shows a modular sorbent cartridge with three modules with an optional bypass line connected to another component such as a recharger.
FIG. 13 shows two modules parallel to each other with recharger connectors.
FIG. 14 shows a three module sorbent cartridge where the first module is in series with the second and third modules, wherein the second and third modules are parallel to each other, and includes four recharger connectors.
FIG. 15 shows a three module sorbent cartridge where the first module is in series with the second and third modules, wherein the second and third modules are parallel to each other.
FIG. 16 shows a four module sorbent cartridge with two sets of parallel modules connected to eight recharger connectors and a bypass line.
FIG. 17 shows a six module sorbent cartridge with three sets of parallel modules, twelve recharger connectors and a bypass line.
FIG. 18 shows a single module from a modular sorbent cartridge with a bypass line, and two wash lines each divided into gas and fluid wash lines.
FIG. 19 shows the use of a sorbent cartridge in a controlled compliance dialysis circuit.
FIG. 20 shows two modules parallel to each other with a single recharger connector.
FIG. 21 shows a three module sorbent cartridge where the first module is in series with the second and third modules, and wherein the second and third modules are parallel to each other, and includes one recharger connector.
FIG. 22 shows a four module sorbent cartridge with two sets of parallel modules, wherein each set connects to a recharger connector and a bypass line.
FIG. 23 shows a six module sorbent cartridge with three sets of parallel modules, wherein each set connects to a recharger connector and a bypass line.
FIG. 24 shows a three module sorbent cartridge where the first module is in series with the second and third modules, and wherein the second and third modules are parallel to each other, with an optional bypass line.
FIG. 25 shows a three module sorbent cartridge where the first module is in series with the second and third modules, and wherein the second and third modules are parallel to each other, with a recharger connector and bypass line.
FIG. 26 shows a single module from a modular sorbent cartridge with a bypass line, a recharger connector, and wash lines adapted to move both liquid and gas.

### DETAILED DESCRIPTION OF THE INVENTION

Unless defined otherwise, all technical and scientific terms used herein generally have the same meaning as commonly understood by one of ordinary skill in the relevant art.

The articles "a" and "an" are used herein to refer to one or to more than one (*i.e.,* to at least one) of the grammatical object of the article. By way of example, "an element" means one element or more than one element.

The term "before," as used when referring to relative positions of components of a dialysis system, refers to an upstream position in the normal operational flow direction of the dialysis system. The term "after" refers to a downstream position in the normal operational flow direction of the dialysis system.

"Blow out" refers to the process of passing a gas through a connection line or a module.

"Bypass line" refers to a line, connected to the main line, through which fluid or gas may alternatively flow.

The term "cartridge" refers to any container designed to contain a powder, liquid, or gas made for ready connection to a device, structure, system, flow path, or mechanism. The container can have one or more compartments. Instead of compartments, the container can also be comprised of a system of two or more modules connected together to form the cartridge wherein the two or more modules once formed can be connected to a device, structure, system, flow path, or mechanism.

The term "cation concentrate reservoir" refers to an object having or holding a substance that is comprised of at least one cation, for example calcium, magnesium, or potassium ions.

The term "cation infusate source" refers to a source from which cations can be obtained. Examples of cations include, but are not limited to, calcium, magnesium and potassium. The source can be a solution containing cations or a dry composition that is hydrated by the system. The cation infusate source is not limited to cations and may optionally include other substances to be infused into a dialysate or replacement fluid, non-limiting examples can be glucose, dextrose, acetic acid and citric acid.

The term "comprising" includes, but is not limited to, whatever follows the word "comprising." Thus, use of the term indicates that the listed elements are required or mandatory but that other elements are optional and may or may not be present.

A "connector" as used herein forms a fluid connection between two components wherein fluid or gas can flow from one of the components, through the connector, to another component. The connector provides for a fluid connection in its broadest sense and can include any type of tubing, fluid or gas passageway, or conduit between any one or more components of the invention.

The term "consisting essentially of' includes whatever follows the term "consisting essentially of' and additional elements, structures, acts or features that do not affect the basic operation of the apparatus, structure or method described.

The term "consisting of' includes and is limited to whatever follows the phrase "consisting of." Thus, the phrase indicates that the limited elements are required or mandatory and that no other elements may be present.

The term "container" as used herein is a receptacle that may be flexible or inflexible for holding any fluid or solid, such as for example a spent dialysate fluid, or a sodium chloride or sodium bicarbonate solution or solid, or urease, or urease/alumina, or the like.

The terms "controlled compliance" and "controlled compliant" describe the ability to actively control the transfer of fluid volume into or out of a compartment, flow path or circuit. In certain embodiments, the variable volume of fluid in a dialysate circuit or controlled compliant flow path expands and contracts via the control of one or more pumps in conjunction with one or more reservoirs. The volume of fluid in the system is generally constant (unless additional fluids are added to a reservoir from outside of the system) once the system is in operation if patient fluid volume(s), flow paths, and reservoirs are considered part of the total volume of the system (each individual volume may sometimes be referred to as a fluid compartment). The attached reservoirs allow the system to adjust the patient fluid volume by withdrawing fluid and storing the desired amount in an attached control reservoir and/or by providing purified and/or rebalanced fluids to the patient and optionally removing waste products. The terms "controlled compliance" and "controlled compliant" are not to be confused with the term "non-compliant volume," which simply refers to a vessel, conduit, container, flow path, conditioning flow path or cartridge that resists the introduction of a volume of fluid after air has been removed from a defined space such as a vessel, conduit, container, flow path, conditioning flow path or cartridge. In one embodiment, the controlled compliant system can move fluids bi-directionally. In certain cases, the bi-directional fluid movement can be across a semi-permeable membrane either inside or outside a dialyzer. The bi-directional fluid flow can also occur across, through, or between vessels, conduits, containers, flow paths, conditioning flow paths or cartridges of the invention in selected modes of operation. The term "moving fluid bi-directionally" as used in connection with a barrier, such as a semi-permeable membrane, refers to the ability to move fluid across the barrier in either direction. "Moving fluid bi-directionally" also can apply to the ability to move fluid in both directions in the flow path or between a flow path and reservoir in a controlled compliant system.

The terms "controlled compliant flow path," "controlled compliant dialysate flow path" and "controlled compliant solution flow path" refer to flow paths operating within a controlled compliant system having the characteristic of controlled compliance, or of being controlled compliant as defined herein.

A "controller," "control unit," "processor," or "microprocessor" is a device which monitors and affects the operational conditions of a given system. The operational conditions are typically referred to as output variables of the system wherein the output variables can be affected by adjusting certain input variables.

A "control pump" is a means capable of moving fluid through a system at a specific rate. The term "control pump" can include for example an "ultrafiltrate pump," which is a pump that is operable to pump fluid bi-directionally to actively control the transfer of fluid volume into or out of a compartment or circuit.

A "control system" consists of combinations of components that act together to maintain a system to a desired set of performance specifications. The control system can use processors, memory and computer components configured to interoperate to maintain the desired performance specifications. The control system can also include fluid or gas control components and solute control components as known within the art to maintain the performance specifications.

A "control valve" is a valve for controlling the movement of a liquid or a gas. When the control valve directs the movement of gas, the "control valve" can open or close to regulate the movement of gas from a high pressure gas source to a lower pressure.

A "degasser" is a component that is capable of removing dissolved and undissolved gasses from fluids.

The term "detachable" or "detached" relates to any component of the present invention that can be separated from a system, module, cartridge or any component of the invention. "Detachable" can also refer to a component that can be taken out of a larger system. In certain instances, the components can be detached with minimal time or effort, but in other instances can require additional effort. The detached component can be optionally reattached to the system, module, cartridge or other component. A detachable module can often be part of a reusable module as defined herein.

"Dialysate" is the fluid that passes through the dialyzer on the side of the dialysis membrane that is opposite to the fluid (e.g. blood) that is being dialyzed.

"Dialysis" is a type of filtration, or a process of selective diffusion through a membrane. Dialysis removes solutes of a specific range of molecular weights via diffusion through a membrane from a fluid to be dialyzed into a dialysate. During dialysis, a fluid to be dialyzed is passed over a filter membrane, while dialysate is passed over the other side of that membrane. Dissolved solutes are transported across the filter membrane by diffusion between the fluids. The dialysate is used to remove solutes from the fluid to be dialyzed. The dialysate can also provide enrichment to the other fluid.

The term "dialyzer" refers to a cartridge or container with two flow paths separated by semi-permeable membranes. One flow path is for blood and one flow path is for dialysate. The membranes can be in the form of hollow fibers, flat sheets, or spiral wound or other conventional forms known to those of skill in the art. Membranes can be selected from the following materials of polysulfone, polyethersulfone, poly(methyl methacrylate), modified cellulose, or other materials known to those skilled in the art.

"Disposable" refers to a component that is to be removed from the system and not reused.

The term "extracorporeal," as used herein generally means situated or occurring outside the body.

The term "extracorporeal circuit" or "extracorporeal flow path" refers to a fluid pathway incorporating one or more components such as but not limited to conduits, valves, pumps, fluid connection ports or sensing devices configured therein such that the pathway conveys blood from a subject to an apparatus for hemodialysis, hemofiltration, hemodiafiltration or ultrafiltration and back to the subject.

The terms "extracorporeal flow path pump" and "blood pump" refer to a device to move or convey fluid through an extracorporeal circuit. The pump may be of any type suitable for pumping blood, including those known to persons of skill in the art, for example peristaltic pumps, tubing pumps, diaphragm pumps, centrifugal pumps, and shuttle pumps.

"Flow" refers to the movement of a fluid, a gas, or both.

A "flow sensing apparatus" or "flow measuring apparatus" is an apparatus capable of measuring the flow of liquid or gas within a specific area.

A "fluid" is a subset of the phases of matter and can include liquids, gases, plasmas and, to some extent, plastic solids. Notably, a liquid, as used herein, can therefore also have a mixture of gas and liquid phases of matter.

The term "fluid communication" refers to the ability of fluid or gas to move from one component or compartment to another within a system or the state of being connected, such that fluid or gas can move by pressure differences from one portion that is connected to another portion.

The term "fluidly connectable" refers to the ability of providing for the passage of fluid or gas from one point to another point. The two points can be within or between any one or more of compartments, modules, systems, components, and recharger, all of any type.

"Infusate" is a solution of one or more salts for the adjustment of the composition of a dialysate.

The term "in-line" refers to state in which a module or set of modules is fluidly connected to dialysis machine, dialysis flow path or dialysis circuit. Dialysis can be on-going, paused or stopped during the in-line state wherein in-line only refers to the state of the modules being fluidly connected to the dialysis machine, dialysis flow path or dialysis circuit.

"Module" refers to a discreet component of a system. Each of the modules can be fitted to each other to form a system of two or more modules. Once fitted together, the modules can be in fluid connection and resist inadvertent disconnection. A single module can represent a cartridge to be fitted to a device or mechanism if the module is designed to contain all the necessary components for an intended purpose such as a sorbent for use in dialysis. In such a case, the module can be comprised of one or more compartments within the module. Alternatively, two or more modules can form a cartridge to be fitted to a device or mechanism where each module individually carries separate components but only when connected together contain in summation all the necessary components for an intended purpose such as a sorbent for use in dialysis. A module can be referred to as a "first module," "second module," "third module," *etc.* to refer to any number of modules. The designation of "first," "second," "third," etc. does not refer to the respective placement of the module in the direction of fluid or gas flow, and merely serves to distinguish one module from another unless otherwise indicated.

The term "non-reusable" refers to a component that cannot be reused in the component's current state. In certain instances, the term non-reusable can include the concept of being disposable, but is not necessarily limited to just being disposable.

The term "off-line" refers to a state in which a module or set of modules is fluidly disconnected from a dialysis machine, dialysis flow path or dialysis circuit. Dialysis can be on-going, paused or stopped during the off-line state wherein off-line only refers to the state of the modules being fluidly disconnected from the dialysis machine, dialysis flow path or dialysis circuit. The off-line state can also include a process whereby the module or set of modules is being recharged as defined herein.

The term "online" refer to state in which a module or set of modules is fluidly connected to dialysis machine, dialysis flow path or dialysis circuit. Dialysis can be on-going, paused or stopped during the online state wherein online only refers to the state of the modules being fluidly connected to the dialysis machine, dialysis flow path or dialysis circuit.

An "operational line" or "line" is a passageway, conduit or connector that directs fluid or gas in a path used while the system is in operation.

The terms "pathway," "conveyance pathway," "fluid flow path," and "flow path" refer to the route through which a fluid or gas, such as dialysate or blood, travels.

A "photocell" is a sensor capable of measuring light or other electromagnetic radiation.

The terms "pressure meter" and "pressure sensor" refer to a device for measuring the pressure of a gas or liquid in a vessel or container.

A "pressure valve" is a valve wherein, if the pressure of the fluid or gas passing the valve reaches a certain level, the valve will open to allow fluid or gas to pass through.

The term "pump" refers to any device that causes the movement of fluids or gases by the application of suction or pressure.

A "push-on fitting" is a fitting for connecting two components wherein the components may be connected by applying pressure to the base of the fitting attached to the components.

A "quick connect fitting" is a fitting for connecting two components wherein the male portion of the fitting contains flexible flanges extending outward with a portion on the end of the flange extending further outward, and the female portion of the fitting contains an internal ridge so that when connected, the outward extending portion of the flange sits under the ridge. By applying pressure, the flexible flange can be forced inward, past the ridge, enabling easy removal.

A "recharger" is a component that is capable of recharging spent sorbent material to or near its original state or usable capacity. A recharger may be part of the dialysis system, or may be separate from the rest of the system. If the recharger is separate from the rest of the dialysis system, the term may include a separate facility where the spent sorbent material is sent to be returned to, or near, its original state. A "recharger connector" or "recharger node" is a connector that fluidly connects a recharger to another component.

"Recharging" to restore the functional capacity of the sorbent material so as to put the sorbent material back into a condition for reuse or use in a new dialysis session. In some instances, the total mass, weight and/or amount of "rechargeable" sorbent materials remain the same. In some instances, the total mass, weight and/or amount of "rechargeable" sorbent materials change. Without being limited to any one theory of invention, the recharging process may involve exchanging ions bound to the sorbent material with different ions, which in some instances may increase or decrease the total mass of the system. However, the total amount of the sorbent material will in some instances be unchanged by the recharging process. Upon a sorbent material undergoing "recharging," the sorbent material can then be said to be "recharged."

The term "recyclable" refers to material that can be reused.

"Reusable" refers in one instance to a material that can be used more than one time, optionally with treatment of any type of the material between uses. For example, a material and a solution can be reused. In one instance, reusable can also refer to a cartridge, as used herein, that contains a material that can be recharged by recharging the material(s) contained within the cartridge.

A "sensor" is a component capable of determining the states of one or more variables in a system.

"Sorbent cartridge" refers to a cartridge that can contain one or more sorbent materials. The cartridge can be connected to a dialysis flow path. The sorbent materials in the sorbent cartridge are used for removing specific solutes from solution, such as urea. The sorbent cartridge can have a single compartmental design wherein all sorbent materials necessary for performing dialysis are contained within the single compartment. Alternatively, the sorbent cartridge can have a modular design wherein the sorbent materials are dispersed across at least two different modules, which can be connected to form a unitary body. Once the at least two modules are connected together, the connected modules can be referred to as a sorbent cartridge, which can be fitted to a device or mechanism. It will be understood that when a single module contains all the sorbent materials necessary for performing dialysis, the single module can be referred to as a sorbent cartridge.

"Sorbent materials" are materials capable of removing specific solutes from solution, such as urea.

"Spent dialysate" is a dialysate that has contacted with blood through a dialysis membrane and contains one or more impurity, waste species, or waste substance, such as urea.

The term "substantially inflexible volume" refers to a three-dimensional space within a vessel or container that can accommodate a maximum amount of non-compressible fluid and resists the addition of any volume of fluid above the maximum amount. The presence of a volume of fluid less than the maximum amount will fail to completely fill the vessel or container. Once a substantially inflexible volume has been filled with a fluid, removal of fluid from that volume will create a negative pressure that resists fluid removal unless fluid is added and removed simultaneously at substantially equal rates. Those skilled in the art will recognize that a minimal amount of expansion or contraction of the vessel or container can occur in a substantially inflexible volume; however, addition or subtraction of a significant volume of fluid over a maximum or minimum will be resisted.

"Tap water" refers to water obtained through piping from a water supply without additional treatment.

A "threaded fitting" is a fitting for connecting two components wherein the male portion has a helical ridge wrapped around a cylinder, and the female portion is a cylindrical hole with internal helical ridges so that when the male portion is screwed into the female portion the two components are locked together.

A "twist-lock fitting" is a fitting for connecting two components wherein the male portion of the fitting contains a head with a length exceeding its width, the female portion of the fitting is a hole with a length that exceeds its width and is larger than the male portion, so that when the male portion is inserted into the female portion and either portion is twisted the two components become locked together.

"Uremic toxins" are toxins carried in the blood supply normally removed in the kidneys.

A "valve" is a device capable of directing the flow of fluid or gas by opening, closing or obstructing one or more pathways to allow the fluid or gas to travel in a particular path. One or more valves configured to accomplish a desired flow can be configured into a "valve assembly."

A "wash line" is a line that directs fluid between a recharger and a module.

The term "waste fluid" refers to any fluid that does not have a present use in the operation of the system. Non-limiting examples of waste fluids include ultrafiltrate, or fluid volume that has been removed from a subject undergoing a treatment, and fluids that are drained or flushed from a reservoir, conduit or component of the system.

The term "waste species," "waste products," "waste," or "impurity species" refers to any molecular or ionic species originating from the patient or subject, including metabolic wastes, molecular or ionic species including nitrogen or sulfur atoms, mid-weight uremic wastes and nitrogenous waste. Waste species are kept within a specific homeostasis range by individuals with a healthy renal system.

The term "water source" refers to a source from which potable or not potable water can be obtained.

### Sorbent Dialysis

Sorbent dialysis allows for dialysis with a small volume of dialysate, creating many advantages. In sorbent dialysis, spent dialysate, containing toxins removed from the blood of the patient, is passed through a sorbent cartridge. The sorbent cartridge of the invention can contain sorbent materials that selectively remove specific toxins from the spent dialysate, either completely or by replacing them with non-toxic material. This process converts the spent dialysate into clean dialysate, which can then be redirected back to the dialyzer.

Modular sorbent cartridges, wherein each module contains select sorbent materials, can be useful in sorbent dialysis. This modular design critically allows for certain portions of the sorbent cartridge to be discarded, refilled, recycled or recharged. In any embodiment of the first, second or third aspects of the invention, the sorbent materials can be structured into layers and/or intermixed. In particular, the modules can have the sorbent materials either intermixed or in layers wherein any combination of intermixed and layered modules can be used interchangeably together.

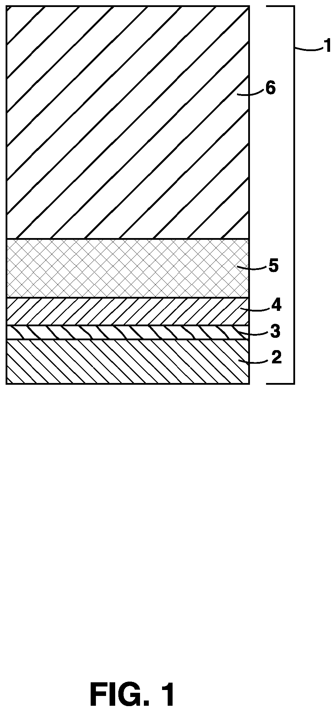

One non-limiting, exemplary sorbent cartridge is shown in FIG. 1. Spent dialysate can flow from the bottom of the sorbent cartridge 1 to the top of the cartridge. The first sorbent material the spent dialysate (or fluid) contacts can be activated carbon 2. Activated carbon will remove nonionic toxins from the fluid by adsorption. Creatinine, glucose, uric acid, P2-microglobulin and other non-ionic toxins, except urea, can be adsorbed onto the activated carbon, removing those toxins from the fluid. Other non-ionic toxins will also be removed by the activated carbon. The dialysate (or fluid) then continues through the sorbent cartridge to the hydrous zirconium oxide layer 3. The hydrous zirconium oxide layer can remove phosphate and fluoride anions, exchanging them for acetate anions. The fluid can continue to move through the sorbent cartridge into the alumina/urease layer 4. Urease can catalyze the reaction of urea to form ammonia and carbon dioxide. The result of this is the formation of ammonium carbonate. The phosphate anions present in the fluid can also be exchanged for hydroxide ions on the alumina. As the fluid continues through the sorbent cartridge, it reaches alumina layer 5. Alumina layer 5 can remove any remaining phosphate ions from the fluid and help retain urease within the sorbent cartridge, and in certain configurations this layer can exchange urea for ammonium and other components. The last layer through which the fluid travels can be zirconium phosphate layer 6. In the zirconium phosphate layer **6,** ammonium, calcium, potassium and magnesium cations can be exchanged for sodium and hydrogen cations. Ammonium, calcium, potassium and magnesium ions all preferentially bind to the zirconium phosphate, releasing the hydrogen and sodium ions originally present in the layer. The ratio of sodium to hydrogen ions released depends on the ratio originally present in the zirconium phosphate layer **6,** and is therefore controllable. The result of the fluid passing through the sorbent cartridge is that the fluid can be regenerated and form clean dialysate that can be safely passed back through a dialyzer to a patient. In any embodiment of the first, second or third aspects of the invention, potassium, calcium and magnesium can be added to the clean dialysate to replace any ions which were removed by the sorbent cartridge. Those ions can be added and/or controlled via an infusate system that can be positioned on the section of the fluid flow path after the sorbent cartridge.

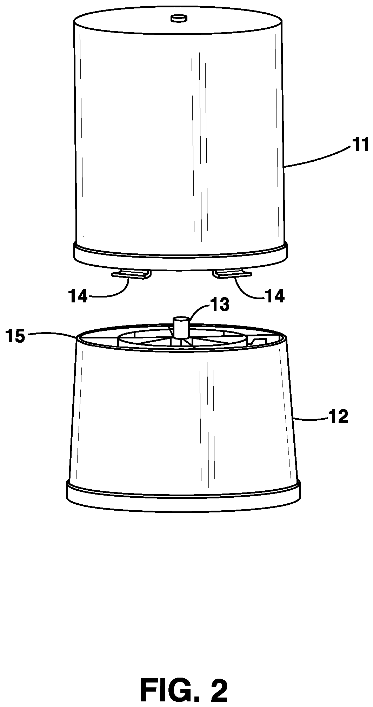

Given the cost of the sorbent cartridges and sorbent materials, it would be advantageous if parts of the cartridge could be reused or recharged. The present invention relates to a sorbent cartridge which includes at least one reusable module, as defined in the claims. As shown in FIG. 2, a reusable module **11** can be fluidly attached to a non-reusable module **12** by a connector **13** with the use of latches **14.** The latches **14** can be integrally formed as part of the reusable module **11,** or non-reusable module **12.** Alternatively, they may be a separate component that must be attached to the module **11.** The latches **14** operate to hold reusable module **11** and non-reusable module **12** together, and may be made out of any material known to those of ordinary skill. The latch members **14** can be mated to an annular connection ring **15** disposed on the circumference of module **12.** One or more engagement members can be disposed inside the annular connection ring **15** to engage the latches **14** when positioned relative to each other using a radial motion. Such engagement can cause a rigid connection between the reusable module **11** and the non-reusable module **12.** Other known locking or fastening mechanisms known to those of ordinary skill that can effectuate rapid and effective connections between two components are contemplated by the invention. Although only cylindrical modules are shown, it will be understood that modules of any shape such as rectangular, conical, triangular, etc. are contemplated by the present invention with a correspondent fastening mechanism. It will be understood that different combinations of reusable and non-reusable modules can be combined together. In any embodiment of the first, second or third aspects of the invention, both modules may be reusable or both may be non-reusable. Moreover, any one of the modules can be detachable from each other or from a casing forming the body of the sorbent cartridge.

The modules can be standardized components that are interchangeable with other modules and easily assembled. For example, the latches **14** in FIG. 2 allow for a simple, twist-lock between two modules. The twist lock allows for the modules to be connected to each other by an easy and rapid manual motion not requiring complex maneuvering of the modules. The connection, once made, can be resistant to inadvertent disengagement, but can also be readily disengaged when desired with a similar easy and rapid manual manipulation. For example, a force applied on the outside periphery of the modules near the latch, e.g. squeezing the module, can cause the latch member **14** to disengage from the engagement members. In other examples, the modules can be disengaged by simply rotating the modules relative to each other.

In any embodiment of the invention, each module can function as a sorbent cartridge independently. In any embodiment of the invention, at least two modules can cooperate together when engaged to each other using, for example the latches **14** in FIG. 2 and being fluidly connected together to function as a sorbent cartridge. The advantage of such a modular design as described herein is that different sorbent materials can be dispersed between the at least two modules to allow for any particular sorbent or combination of sorbent materials to be detachable from a sorbent cartridge.

In any embodiment of the invention, the connector **13** can be formed as part of the reusable module **11** and non-reusable module **12** and need not be a separate component that must be attached to the module **12.** Rather, the connector **13** can be molded as part of the reusable module **11** and non-reusable module **12.** The connector can be a combination of female and male connectors on a module. For example, a female connector can be disposed on one module, and a male connector on the other to form one connector **13** (not shown). In any embodiment of the invention, the connector can be affixed by mechanical means, glued or rigidly interfaced to the modules **11** and **12.** In any embodiment of the invention, the connector **13** allows fluid to flow from the non-reusable module, through the connector, into the reusable module. Alternatively, in any embodiment of the invention, the connector **13** is not a part of either the non-reusable module **12** or reusable module **11** but can be a separate component such as tubing. It will be understood that the connector **13** is defined in its broadest sense and encompasses any fluid connection between two points.

In any embodiment of the invention, one or more fluid connectors can be arranged between any module of the invention, and one or more such fluid connectors can be provided in any of the described configurations herein. For example, a reusable or non-reusable module can have any number of connectors such as 1, 2, 3, 4, 5, or more. The spacing and distribution of the fluid connectors on the module can be positioned to enable and/or increase flow of fluid between the modules. In any embodiment of the invention, the fluid connectors can be spaced equidistant from each other or may be located axially or radially. The sorbent cartridge can also have one or more modules each having any number of fluid connectors. In contrast to known sorbent cartridges having a unitary design in which sorbent materials are arranged in layers without any connectors between such layers, the fluid connectors of the invention allow for controlled fluid or gas flow to any particular sorbent or combination of sorbent materials. The fluid connectors also allow for any particular sorbent or combination of sorbent materials to be detachable from a sorbent cartridge. For example, a detachable module can be constructed with one or more sorbent materials. The detachable module can then be fluidly connected to the sorbent cartridge by fluid connectors. Such a configuration advantageously allows for separate treatment, recycling, or recharging of the sorbent or combination of sorbent materials not possible with known sorbent cartridges. In particular, known sorbent cartridges have all the sorbent materials being formed into layers or a plurality of sorbent materials being mixed without connectors in between such layers of one sorbent material, or mixtures of sorbent materials. It will be understood that the fluid connectors of the invention can be critical because the connectors control the order of sorbent materials to which a fluid or gas is exposed, the delivery of fluid or gas to a particular sorbent or combination of sorbent materials, and the flow and rate of flow of a fluid or gas to various sorbents and combinations of sorbent material.

In any embodiment of the invention, it will be understood that the present invention contemplates at least two modules that fit together, which is distinct from known dialysis systems requiring separate housings containing sorbent materials that do not form a unitary sorbent cartridge for ready attachment or insertion into a dialysis machine. A unitary sorbent cartridge of the present invention contains each one of the sorbent materials described herein including cation and anion exchange resins inside the sorbent cartridge. In other words, the cation and anion exchange resins (or other sorbent materials) are not separated into another housing outside the sorbent cartridge. Although the individual sorbent materials of the present invention may be separated into different detachable and/or reusable modules within the single sorbent cartridge wherein each module is connected by fluid connectors, the single sorbent cartridge design provides reduced size and weight that is not possible with the known dialysis systems having separate housings. The modules, as described herein, can also be further rigidly fixed to each other by latches and engagement members or any fixing or fastening mechanism known to those of ordinary skill in the art. Notably, the sorbent cartridge of the invention can have all of the sorbent materials described herein including cation and anion exchange resins within a single unitary sorbent cartridge for convenient removal, service and monitoring. In particular, the sorbent cartridge can have a single compartmental design wherein all sorbent materials necessary for performing dialysis are contained within a single compartment. The sorbent cartridge can also have a modular design wherein the sorbent materials are dispersed across at least two different modules, which can be connected to form a unitary body. Once the at least two modules are connected together, the connected modules can form a sorbent cartridge to be fitted to a device or mechanism. Advantageously, the present sorbent cartridge can therefore be easier to recycle, recharge, dispose of, service and remove from a dialysis machine. In any embodiment of the invention, the unitary design can also provide for a compact design that can be used in a portable dialysis machine. Further, manufacturability is benefited by the unitary design.

In any embodiment of the invention, the fluid connector can be a quick-connect, twist-lock, push-on, or threaded fitting. Other forms of such connection known to those of ordinary skill in the art are also contemplated by the invention. Additionally, the connector can comprise a length of tubing and a valve assembly. In any embodiment of the invention, the connector can be manually assembled to connect any component or assembly of the invention. The connector can also be used to rigidly connect any one of the modules to a recharger as defined herein when a separate fastening mechanism is not provided.

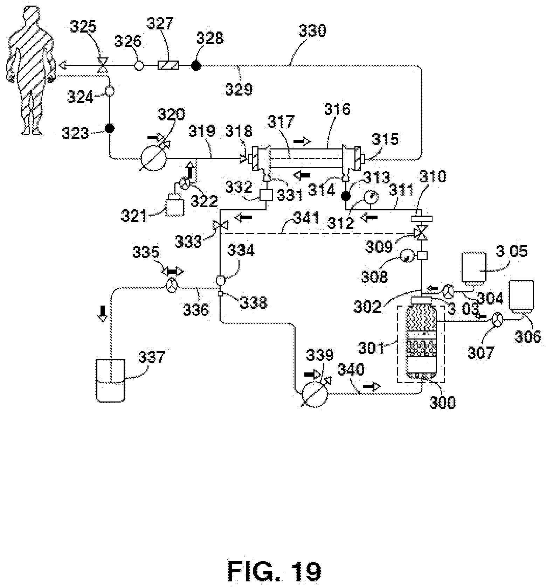

In any embodiment of the at least one module can be in fluid communication with a controlled compliant dialysis circuit. A non-limiting example of a controlled compliant dialysis circuit is shown in FIG. 19 as disclosed in U.S. Patent Application No. 13/565,733. As shown in the controlled compliant dialysis circuit of FIG. 19, the patient's blood is circulated through an extracorporeal circuit **330.** The portion of the extracorporeal circuit **330** that contains drawn blood from the patient can be referred to as the arterial line **319,** which by convention is understood to mean a line for transporting blood from the patient regardless of whether blood is drawn from an artery or vein of the patient. Similarly, the portion that returns blood to the patient can be referred to as the venous line **329.** In any embodiment of the first, second or third aspects of the invention, the arterial line **319** and the venous line **329** connect with one or more veins of the patient. Locomotive power for moving the blood through the extracorporeal circuit **330** is provided by a blood pump **320**, which is typically located along the arterial **319** line. Valve **325** can be placed on venous line **329.** Blood is typically conveyed through the extracorporeal circuit **330** at a rate of 50 to 600 mL/min and can be adjusted by a controller to any required rate suitable for a procedure performed by the invention. Blood pump **320** can be a peristaltic pump, although those skilled in the art will readily understand that other types of pumps can be used including diaphragm pumps, centrifugal pumps, and shuttle pumps. In any embodiment of the first, second or third aspects of the invention, the blood pump **320** conveys blood through the dialyzer **316** where the blood is contacted with a blood side of a high permeability dialysis membrane **317.** Blood enters the dialyzer **316** through a blood inlet **318** and exits through a blood outlet **315.** The pressure of the blood prior to the blood pump **320** is measured by a pressure meter **323** and post dialyzer **316** by a pressure meter **328.** The pressure at pressure meter **323** provides an indication of the adequacy of the blood flow into the circuit where increased vacuum is an indication of a less adequate access flow. The pressure indication at pressure meter **328** can serve to detect obstructions in the venous bloodline. Additional pressure meter **313** can be located after blood outlet **315.** An air trap **327** is placed along the extracorporeal circuit **330** to prevent the introduction of air into the circulatory system of the patient. The air trap **327** is not limited to a particular design. Typical air traps employ a hydrophobic membrane that allows air to be separated from an air- liquid mixture by allowing air to pass through the membrane and retaining water-based fluids. Alternatively the air trap **327** can be run full, where a pressure meter can use a flexible impermeable membrane to transmit pressure pulses to a pressure transducer such that there is no direct air blood interface. Air-fluid detectors **324** and **326** are present to confirm that air is not present in the extracorporeal circuit **330**, and additional air fluid detector **334** can be present in the dialysis circuit **340**. Air fluid detectors **324, 326** and **334** can be ultrasonic sensors that can detect a change in solution density or scattering due to the presence of air or air bubbles.

During the course of conveyance of blood along the extracorporeal circuit **330,** heparin or other anticoagulant is added to the blood to prevent clotting of blood within the dialyzer **316** or blood conveyance pathway/extracorporeal circuit **330.** Heparin or another anticoagulant is added from an anticoagulant container **321** at a metered rate using an anticoagulant pump **322.** The anticoagulant pump **322** can be any pump capable of accurately metering heparin.

Dialysate within the system is conveyed through one of a first dialysate pathway **311** in the dialysate circuit, which carries dialysate to the dialyzer **316,** or a second bypass pathway **341** shown in a dashed line, which serves to bypass the dialyzer **316.** The dialysis circuit can include a pair of quick connectors **338.** The first and second pathways **311** and **341** have one or more conduits for conveying the dialysate. Access to the second bypass pathway **341** is controlled by valve **309**. It is understood by one skilled in the art that three-way valve **309** can be replaced with a two-way valve with the same result to control the flow through the dialyzer **316** or bypass pathway **341.** The first dialysate pathway **311,** the second bypass pathway **341,** and residual volume in the dialyzer **316** including conduits for conveying the dialysate together form a dialysis circuit **340** that houses the circulating volume of the dialysate present in the system. It is understood by one skilled in the art that three-way valve **309** could be replaced with two-way valves with the same result to control the flow through the dialyzer or bypass loop.

Dialysate that is conveyed through the dialyzer **316** on the dialysate side of the dialysis membrane **317** picks up waste produces from the blood, including urea, by diffusion, hemofiltration or hemodiafiltration. Dialysate enters the dialyzer at a dialysate inlet end **314** and exits at an outlet end **331.** The dialysate exiting the dialyzer **316** passes through a blood leak detector **332** that can determine the presence of blood in the dialysate indicating a breach in the dialysis membrane **317.** Flow of dialysate from the dialyzer **316** can be stopped or controlled through the operation of valve **333** as well as to prevent the backup of dialysate into the dialyzer **316.** The dialysate is conveyed through a sorbent cartridge **301** to remove waste products before being re-conveyed through the dialyzer **316.** The dialysate enters the sorbent cartridge **301** at a dialysate inlet end **300** and exits at an outlet end **302.** Refreshed dialysate exiting outlet end **302** of the sorbent cartridge **301** can be monitored by a conductivity meter **308.** Additional conductivity meter **312** can be present. Optionally, the dialysate can be filtered through a microbial filter **310.** An air trap **303** can be positioned before or after outlet end **302** to remove gasses introduced into the dialysate by the sorbent cartridge **301.** The volume of actively circulating dialysate is determined by the total void volume of the conduits and the sorbent cartridge **301** forming the dialysis circuit **340.** The void volumes of the conduits and of the sorbent cartridge **301** forming the dialysis circuit **340** have a non-expandable or substantially inflexible volume.

The total void volume of the conduits having a substantially inflexible volume prevents the passive inflow and outflow of fluid volume due to pressure changes that can occur over the course of treatment. This results in a benefit because not all of the pressure changes during treatment are under precise control by a user or operator. A controlled compliance dialysis circuit is achieved by actively controlling the inflow (influx) and outflow (efflux) of fluid to and from the dialysis circuit **340** and the extracorporeal circuit **330.** In this manner, the volume of fluid crossing the dialysate membrane **317** is under direct control and can be accurately determined.

The controlled compliance dialysis circuit can be accurately controlled to precisely remove or add fluid to the dialysis circuit. Due to the substantially inflexible void volume of the conduits, the sorbent cartridge **301** and other components of the dialysis circuit **340**, the net movement of fluid over any time interval across the dialysate membrane can be accurately controlled by creating a means to accurately introduce or remove fluid from the patient. This capability is used to enhance the convective clearance of the system while controlling the net fluid removed from the patient.

As shown in FIG. 19, the dialysate is moved along the dialysis circuit **340** by a dialysate pump **339.** When the control pump **335** is not operating, fluid along the length of the dialysis circuit **340** flows at a rate determined by the dialysate pump **339.** When the control pump **335** is operating, fluid exiting the dialyzer **316** and traveling toward the conduit **336** is flowing at a rate that is the combination of the rates of the control pump **335** and the dialysate pump **339.** However, the fluid traveling from the entry point of conduit **336** into the dialysis circuit **340** to the dialyzer **316** is traveling at the rate of the dialysate pump **339.** As such, the rate of fluid traveling to the dialyzer **316** is not affected by the operation of the control pump **335.** The dialysate pump can be operated at a rate from about 10 to about 400 mL/min, the specific rate being dependent on the rate of the blood pump **320** at the desired contact time with the dialysis membrane **317** to achieve diffusion of impurities from blood to the dialysate. The rate of the dialysate pump **339** and the blood pump **320** can be controlled by a controller (not shown).

Due to the substantially inflexible void volume of the conduits and the sorbent cartridge **301,** bulk fluid or water is prevented from moving across the membrane **317** from the extracorporeal circuit **330** of the dialyzer **316** to the dialysate circuit **340** of the dialyzer **316.** Specifically, due to the controlled compliant feature of the void volume of the dialysis circuit **340**, water cannot passively move from the extracorporeal side to the dialysate side through the dialysis membrane. In the event of factors that tend to increase pressure on the extracorporeal side of the dialysis membrane, such as increased blood flow rate or blood viscosity, pressure across the membrane will automatically be equalized due to the limited volume of the dialysis circuit **340** and the non-compressible nature of the dialysate. In the event of factors that tend to increase pressure on the dialysate side of the dialysis membrane **317,** such as increased dialysis flow rate, net movement of water from the dialysis circuit **340** to the extracorporeal circuit **330** is prevented by a vacuum that would form in the dialysate circuit **340** in the event of such a movement. Since the dialyzer can be a high flux type, there is some fluid flux back and forth across the dialyzer membrane due to the pressure differential on the blood and dialysate sides of the membrane. This is a localized phenomenon due to the low pressure required to move solution across the membrane and is called backfiltration, however results in no net fluid gain or loss by the patient.

Using the controlled compliance dialysis circuit described herein, net movement of water across the dialysis membrane occurs under active control rather than passively due to pressure differences that develop across the dialysis membrane due to normal operations. A control pump **335** is present and accesses the controlled compliance dialysis circuit **340** through a conduit **336.** In any embodiment of the first, second or third aspects of the invention, the conduit **336** joins with the controlled compliance dialysis circuit **340** at a point downstream from the dialyzer **316.** The control pump **335** can be operated in an influx direction that moves fluid from a control reservoir **337** to the controlled compliance dialysis circuit **340** or in an efflux direction that moves fluid from the controlled compliance dialysis circuit **340** into the control reservoir **337.** Due to the substantially inflexible volume of the dialysis circuit **340**, volume added to the controlled compliance dialysis circuit when the control pump **335** operates in the influx direction causes net movement of fluid from the dialysate side of the dialysis membrane **317** to the extracorporeal side of the dialysis membrane **317.** When the control pump **335** is operated in the efflux direction, fluid is drawn from the extracorporeal side of the dialysis membrane into the controlled compliance dialysis circuit. In any embodiment of the first, second or third aspects of the invention, the control pump **335** can be operated at a rate from 0 to about 500 mL/min in either direction.

An infusate pump **304** is used to add a cation infusate **305** into the hemofiltration circuit **340** to generate a fluid having a proper physiological composition to serve as a replacement fluid for introduction into the extracorporeal circuit **330.** A bicarbonate solution in a container **306** can further be added by a pump **307** to maintain a physiological pH in the fluid prior to introduction to the extracorporeal circuit.

It will be understood that the connector provides for a fluid connection in its broadest sense and can include any type of tubing, fluid or gas passageway, or conduit between any one or more components of the invention.

The sorbent material within the module can be recharged by passing a solution containing the proper solutes through the layers of the sorbent module. To recharge the sorbent modules in-line, the modules may be connected by wash lines to rechargers, which contain solutions for recharging the modules.

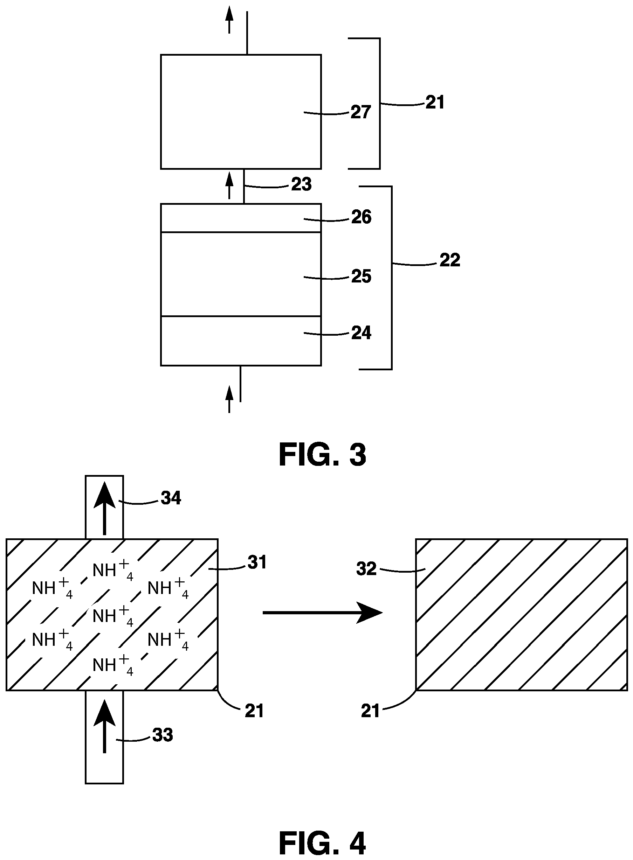

One embodiment of the modular sorbent cartridge of the invention is shown in FIG. 3. The non-reusable module **22** of the sorbent cartridge can contain layers of activated carbon **24,** alumina/urease **25,** and hydrous zirconium oxide **26.** The reusable module **21** contains zirconium phosphate **27.** In any embodiment of the invention, the term non-reusable can refer to the components in a cartridge, and in other embodiments, the term can refer to both the components in the cartridge and the cartridge itself.

After dialysis is complete, the zirconium phosphate layer **27** can contain ammonium, calcium, potassium and magnesium. The module **21** containing the zirconium phosphate may be removed, and the zirconium phosphate can be recharged. The reusable module **21** can be disconnected from the connector **23** connecting the reusable module **21** to the non-reusable module **22,** bypass line and/or wash line. The reusable module **21** is then removed from the modular sorbent cartridge. This module **21** can then be recharged, discarded and replaced, or alternatively, the sorbent material within the module can be removed and refilled. It will be understood that any one of the materials used in the present invention can be used multiple times. In such instances of multi-session use, the number of sessions in which one component can be used, can be the same or different from the number of sessions in which another component can be used. In one non-limiting example of the invention, a module containing urease may be used two times while another module containing zirconium phosphate can be used three times. In any embodiment of invention, the module containing urease can be used three times, and the module containing zirconium phosphate used two times. It will be understood that there is no limitation on the numbers of uses for any multi-session use module as compared to another module used in the sorbent cartridge.

A method of recharging the zirconium phosphate module is shown in FIG. 4. Wash fluid **33,** containing sodium and hydrogen ions, can be passed through the reusable module **21,** containing the used zirconium phosphate **31** with bound ammonium ions. This causes an exchange of ions, wherein hydrogen and sodium ions can replace the ammonium ions on the zirconium phosphate **31.** The waste fluid exiting the module **34** thus contains the freed ammonium ions with excess sodium and hydrogen ions. This process creates a recharged zirconium phosphate layer **32,** containing sodium and hydrogen ions for a subsequent dialysis. A recharger can be used to recharge spent sorbent material wherein the recharger contains fluid capable of restoring spent sorbent material to, or near, its original state or usable capacity.

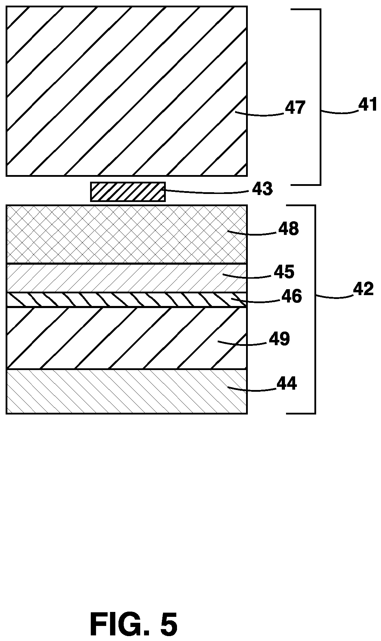

Because calcium and magnesium ions may be more difficult to remove from the zirconium phosphate, and therefore the zirconium phosphate may be more difficult to recharge, it may be advantageous to remove the calcium and magnesium in the first, non-reusable, module so that none of those ions need to be removed in the reusable zirconium phosphate module. Such an embodiment is shown in FIG. 5. Spent dialysate enters the first, non-reusable module **42** where the dialysate can first flow through a layer of activated carbon **44** to remove non-ionic uremic toxins. The dialysate can then enter into a first layer of zirconium phosphate **49.** The zirconium phosphate layer **49** can remove the calcium, magnesium and potassium from the fluid. Next the fluid enters the hydrous zirconium oxide layer **46,** which removes the phosphate anions and exchanges them with acetate anions. The fluid can then enter the urease layer **45** and alumina layer **48,** where the urea is converted to ammonium carbonate and any remaining phosphate ions are removed. In any embodiment of the non-reusable module, any arrangement of the activated carbon, zirconium phosphate, hydrous zirconium oxide layer, and urease and alumina layer is contemplated. For example, the dialysate can first flow through a first layer of zirconium phosphate, activated carbon, then the hydrous zirconium oxide layer, and then enter the urease layer and alumina layer. Alternatively, the dialysate can first flow through the hydrous zirconium oxide layer, then a first layer of zirconium phosphate, the activated carbon, then enter the urease layer and alumina layer. Still further, the dialysate can first flow through the urease layer and alumina layer, then the hydrous zirconium oxide layer, then a first layer of zirconium phosphate, and then the activated carbon. The fluid then flows through the connector **43,** and into the second, reusable, sorbent module **41.** The sorbent module **41** can contain zirconium phosphate **47.** Zirconium phosphate layer **47** can exchange the ammonium ions for sodium and hydrogen. Because the calcium, magnesium and potassium ions have already been removed by the first zirconium phosphate layer **49,** the zirconium phosphate layer **47** will not pick up those ions. After dialysis, the second module **41** will only contain zirconium phosphate bound to ammonium ions. As such, the sorbent may be easier to recharge.

In embodiments where one module contains zirconium phosphate and ion-exchange resin, or zirconium phosphate and hydrous zirconium oxide, the module may be recharged in the same manner. The activated carbon layer of a reusable module can be recharged by passing a heated water solution through the module. The alumina/urease layers can be recharged by first passing heated water, or the solutions described above for recharging zirconium phosphate, through the layer, and then passing a solution containing urease through the alumina/urease layer.

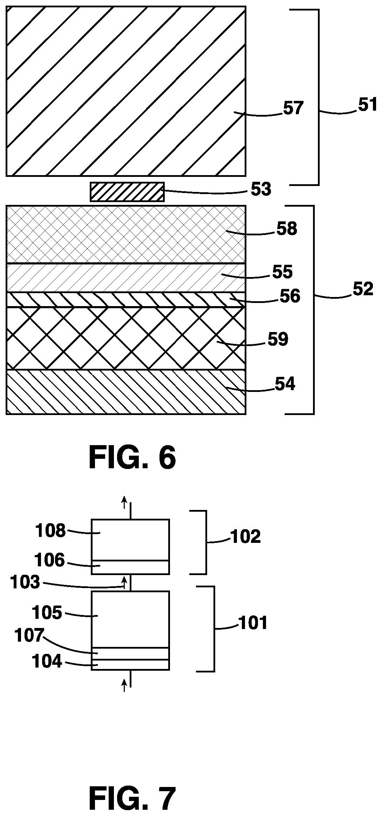

Another non-limiting embodiment is illustrated in FIG. 6. Spent dialysate can enter the first, non-reusable, module 52 where it first flows through a layer of activated carbon **54** to remove non-ionic uremic toxins. The spent dialysate then enters into a layer of ion exchange resin **59.** The ion exchange resin layer **59** can remove the calcium, magnesium and potassium from the fluid. Next the spent dialysate can enter the hydrous zirconium oxide layer **56,** which removes the phosphate anions and exchanges them with acetate anions. The spent dialysate then enters the urease layer **55** and alumina layer **58,** where the urea is converted to ammonium carbonate and any remaining phosphate ions are removed. Any arrangement of the activated carbon, ion exchange resin, hydrous zirconium oxide layer, and urease and alumina layer is contemplated. For example, the dialysate can first flow through an ion exchange resin, activated carbon, then the hydrous zirconium oxide layer, and then enter the urease layer and alumina layer. Alternatively, the dialysate can first flow through the hydrous zirconium oxide layer, then the ion exchange resin, the activated carbon, then enter the urease layer and alumina layer. Still further, the dialysate can first flow through the urease layer and alumina layer, then the hydrous zirconium oxide layer, then the ion exchange resin, and then the activated carbon. The fluid can then flow through the connector **53,** and into the second, reusable, sorbent module **51.** The sorbent module **51** contains zirconium phosphate **57.** The zirconium phosphate layer **57** can exchange the ammonium ions for sodium and hydrogen. Because the calcium, magnesium and potassium ions have already been removed by the ion exchange resin layer **59,** the zirconium phosphate layer **57** will not pick up those ions. Alternatively, the ion-exchange resin **59** may be selected to only remove the calcium and magnesium ions, such as by using a chelating ion exchange resin. This can allow use of less of the ion exchange resin. If such a resin is used, the potassium will be removed by the zirconium phosphate **57.** Potassium can be easier to remove from zirconium phosphate than calcium or magnesium. In any embodiment, the sorbent materials in each module may be intermixed as opposed to being arranged in layers.

One skilled in the art will recognize that different combinations of sorbent materials in both the reusable and non-reusable modules of the sorbent cartridge can be used without being beyond the scope of this invention. The sorbent materials described herein can be mixed together in any combination as shown in the specific embodiments of the invention.

In any embodiment, the sorbent cartridge can be removed from a dialysis system. The sorbent cartridge once removed can be separated into one or more modules to be recharged, disposed of, or recycled. For example, FIG. 7 shows an embodiment wherein the second module contains both hydrous zirconium oxide and zirconium phosphate. The spent dialysate can enter the first module **101.** The spent dialysate can first pass through the activated carbon layer **104.** The spent dialysate can next pass through a first layer of zirconium phosphate **107,** which removes the potassium, calcium and magnesium from the dialysate. Next the spent dialysate can move through the alumina/urease layer **105.** In any embodiment of the first module, any arrangement of the activated carbon, zirconium phosphate, and urease and alumina layer is contemplated. For example, the fluid can first flow through activated carbon, then enter the urease layer, and then the zirconium phosphate. Alternatively, fluid can first flow through the zirconium phosphate layer, then activated carbon, and then enter the urease layer and alumina layer. Still further, the fluid can first flow through the urease layer and alumina layer, then the zirconium phosphate, and then the activated carbon. The fluid can then pass through the connector **103,** and into the second module **102.** The second reusable module **102** contains a hydrous zirconium oxide layer **106,** and a second zirconium phosphate layer **108,** which removes the ammonium ions from the fluid. After dialysis, the reusable module **102** containing the hydrous zirconium oxide and zirconium phosphate can be recharged, discarded, or the sorbent material removed and new material added. Wash lines may be attached to connector **103** disposed on the reusable module **102** and a second connector positioned after the reusable module **102** wherein the second connector can be positioned thereon or as part of a fluid flow path (not shown).

One skilled in the art will realize that embodiments of the invention can be included that involve the sorbent materials being mixed within the module, as opposed to arranging the materials in layers. Such mixing of the sorbent materials can be performed by interspersing the sorbent materials in a single layer by any method known to those of skill in the art.

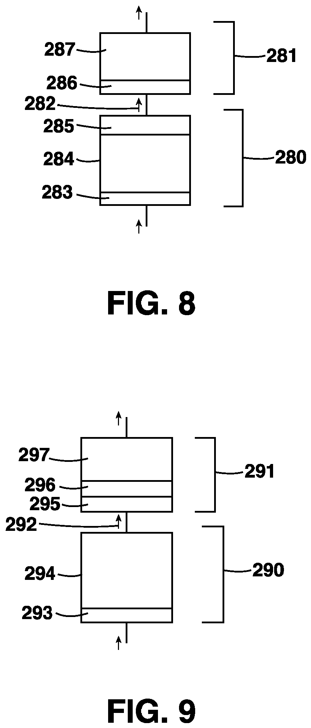

Another non-limiting embodiment is shown in FIG. 8. Spent dialysate can enter the first, non-reusable, module **280** where it first flows through a layer of activated carbon **283** to remove non-ionic uremic toxins. The spent dialysate can then enter into a layer of alumina and urease **284,** where the urea is converted to ammonium carbonate and phosphate ions are removed. Next the fluid can enter the hydrous zirconium oxide layer **285,** which removes the remaining phosphate anions and exchanges them with acetate anions. Any arrangement of the activated carbon, hydrous zirconium oxide layer, and urease and alumina layer is contemplated. For example, the dialysate can first flow through an activated carbon layer, then through the hydrous zirconium oxide layer, and then enter the urease layer and alumina layer. Alternatively, the dialysate can first flow through a hydrous zirconium oxide layer, then the activated carbon layer and then enter the urease layer and alumina layer. Still further, the dialysate can first flow through the urease and alumina layer, then the activated carbon layer, and then enter the hydrous zirconium oxide layer. The dialysate can first flow through a hydrous zirconium oxide layer, then through the alumina and urease layers, and then flow through the activated carbon layer. Alternatively, the dialysate can first flow through the alumina and urease layer, then through the hydrous zirconium oxide layer, and then through the activated carbon layer. The fluid can then flow through the connector **282,** and into the second, reusable, sorbent module **281.** The sorbent module **281** contains an ion exchange resin layer **286,** and a zirconium phosphate layer **287,** which removes the ammonium ions from the fluid. The fluid can first pass through the zirconium phosphate layer and then the ion exchange resin. Alternatively, the sorbent materials in each module may be intermixed as opposed to being arranged in layers. After dialysis, the reusable module **281** containing the zirconium phosphate **287** and ion exchange resin **286** can be recharged, discarded, or the sorbent material removed and new material added.

The hydrous zirconium oxide may be included in the second module as shown in FIG. 9. Spent dialysate can enter the first, non-reusable, module **290** where it first flows through a layer of activated carbon **293** to remove non-ionic uremic toxins. The spent dialysate can then enter into a layer of alumina and urease **294,** where the urea is converted to ammonium carbonate and phosphate ions are removed. The dialysate can first flow through the alumina and urease, and then flow through the activated carbon. The fluid can then pass through connector **292** and into the second, reusable module **291.** The second module **291** contains a layer of hydrous zirconium oxide **295,** a layer of ion exchange resin **296** and a layer of zirconium phosphate **297**. Any arrangement of the hydrous zirconium oxide, ion exchange resin and zirconium phosphate is contemplated. For example, the fluid may first pass through a layer of ion exchange resin, then pass through a layer of hydrous zirconium oxide and then pass through the zirconium phosphate. Alternatively, the fluid may first pass through the ion exchange resin, then pass through the zirconium phosphate and then through the hydrous zirconium oxide. Still further, the fluid may pass through the zirconium phosphate, then through the hydrous zirconium oxide, and then through the ion exchange resin. The fluid can first pass through the hydrous zirconium oxide layer, then through the zirconium phosphate layer, and then through the ion exchange resin. Alternatively, the fluid can first pass through the zirconium phosphate layer, then the ion exchange resin, and then through the hydrous zirconium oxide layer. The sorbent materials in each module can also be intermixed as opposed to being arranged in layers.

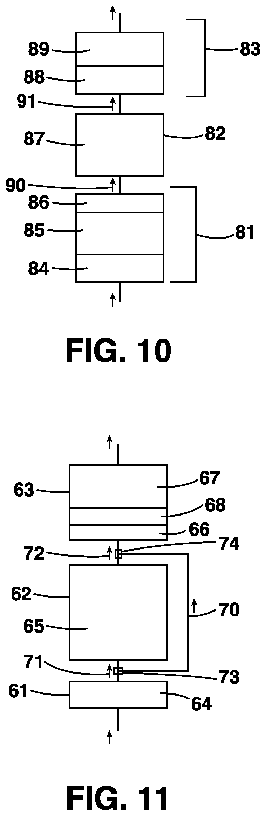

The modular sorbent cartridges are not limited to having two modules. Any number of modules may be utilized. A three module sorbent cartridge is shown in FIG. 10. The first module **81** contains a layer of activated carbon **84,** a layer of alumina/urease **85,** and a layer of hydrous zirconium oxide **86.** The described layers can also be mixed together rather than being provided in layers. Any arrangement of the activated carbon, hydrous zirconium oxide layer, and urease and alumina layer is contemplated. For example, the dialysate can first flow through activated carbon, then the hydrous zirconium oxide layer, and then enter the urease layer and alumina layer. Alternatively, the dialysate can first flow through the hydrous zirconium oxide layer, then the activated carbon, then enter the urease layer and alumina layer. Still further, the dialysate can first flow through the urease layer and alumina layer, then the hydrous zirconium oxide layer, and then the activated carbon. The fluid, after passing through these layers, passes through a first connector **90,** and into the second module **82.** This second module can contain zirconium phosphate **87.** The fluid can then pass through a second connector **91,** and enter a third module **83.** This third module can contain a second layer of zirconium phosphate **88,** and a second layer of activated carbon **89** for final purification before passing out of the sorbent cartridge. In any embodiments of the third module of a three module sorbent cartridge any arrangement of the activated carbon and the second layer of zirconium phosphate are contemplated. For example, the dialysate can first flow through activated carbon and then the second layer of zirconium phosphate. It will be understood that any number of modules can be configured. For a sorbent cartridge having four, five, six, seven, or more modules is contemplated. It will be understood that the described arrangements include not just layers, but also the sorbent materials being intermixed.

As each layer of sorbent material within the modular sorbent cartridge may be recharged, a cartridge is possible where all of the modules are reusable. It is still advantageous to utilize separate modules for the sorbent materials in order to direct the correct recharging solution through the correct module, and because different sorbent materials may need to be replaced more often than others.

Because the ability for the zirconium phosphate layer to bind ammonium ions is finite, while the capacity of the urease layer to break down urea into ammonia is not, the capacity of the zirconium phosphate layer may be exceeded. In such a case, excess ammonium ions can be caused to pass through the sorbent cartridge and remain in the dialysate. To protect patient safety, once ammonia breakthrough has occurred, either the dialysis session can be stopped or at least urease can be prevented from catalyzing the conversion of urea to ammonia.

FIG. 11 shows a three-module sorbent cartridge, that can allow bypass of the alumina/urease layer in the case of ammonia breakthrough. Ammonia breakthrough can occur when the capacity of the zirconium phosphate layer to exchange ammonium ions is exceeded. In the event of ammonia breakthrough, the spent dialysate enters the first module **61,** which contains the activated carbon layer **64.** The spent dialysate then passes through a first connector **71** and by-pass flow valve **73.** In normal operation, the flow valve **73** can be set to allow the fluid to pass into the second module **62.** The second module can contain alumina/urease layer **65,** which catalyzes the breakdown of urea into ammonium ions. The fluid then passes through the second connector **72,** by the second valve **74,** and into the third module **63.** The third module can contain a hydrous zirconium oxide layer **66,** ion-exchange resin **68,** and zirconium phosphate layer **67.** In any embodiment of the third module of a three module sorbent cartridge, any arrangement of the ion-exchange resin, hydrous zirconium oxide layer, and zirconium phosphate layer is contemplated. For example, the dialysate can first flow through ion-exchange resin, then the hydrous zirconium oxide layer, and then enter the zirconium phosphate layer. Alternatively, the dialysate can first flow through the hydrous zirconium oxide layer, then the ion-exchange resin, then enter the zirconium phosphate layer. Still further, the dialysate can first flow through the zirconium phosphate layer, then the hydrous zirconium oxide layer, and then the ion-exchange resin. Again, the described arrangements include not just layers, but also intermixed sorbent materials. After passing through the third module, a regenerated dialysate can exit the sorbent cartridge. In the event of ammonia breakthrough, the first valve **73** can be set to redirect the fluid into bypass line **70.** This line will cause the fluid not to enter the second module **62,** and therefore the urea will not be broken down into ammonia in the alumina/urease layer **65.** The fluid will instead be directed to the second valve **74,** where the fluid enters the second connector **72,** and then the third module **63.** In this way dialysis may continue, while avoiding the creation of ammonia. Either the first valve **73** or the second valve **74** may be optional, and those of skill in the art will recognize that the function can be accomplished with only a single valve if either the first valve **73** or the second valve **74** is a 3-way valve. The valve assembly can also include an access point for a sensor (not shown). The access point can be a portion of the valve assembly wherein a sensor can contact the fluid to take measurement data such as a flow or pressure reading. The form and construction of such access points contemplated by the present invention are those known to one of ordinary skill in the art.

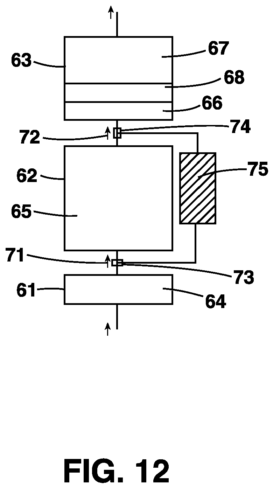

FIG. 12 shows an alternative embodiment of the sorbent cartridge shown in FIG. 11, wherein a first connector **71** and a flow valve **73** bypass flow through the second module **62** to a component **75.** The component **75** can be a recharger used to recharge or clean the second module **62** while attached to the sorbent cartridge. The component **75** can be a container storing a fluid such as a wash fluid or recharging fluid. The component **75** can be a pump for pumping fluid. Upon passing through the component **75,** fluid can return through the second connector **72** via the second valve **74,** and into the third module **63.** The component **75** can be removed after a period of time and fluid allowed to flow to the third module **63** through the second connector **72** and the second valve **74.** The component **75** can be reversibly attached and detached as necessary. In certain embodiments either the first valve **73** or the second valve **74** may be optional, and those of skill in the art will recognize that the function can be accomplished with only a single valve if either the first valve **73** or the second valve **74** is a 3-way valve.

To make use of the modular sorbent cartridge easier, the valve assembly may be operated by a programmable controller or computer system that can be programmed to regulate flow through the valves and into and out of the modules. An optical sensor, photocell or other flow sensing apparatus may detect the flow of fluid through any two points in the sorbent cartridge. For example, in any embodiment of the invention, an optical fluid flow device can be provided for measuring flow wherein the device includes an optical fluid pressure measuring device having sensors positioned in any one of the flow paths between the modules, in the connectors, or in the valve assemblies. Preferably, the sensors will be placed in a passageway defined between the modules. In any embodiment of the invention, the optical fluid sensors can be connected to an interferometer associated with an opto-electronic demodulator which has an output signal representing the differential pressure between the two sensed areas. In any embodiment of the invention, a flow sensing apparatus can have a flow-responsive element projecting into a fluid flow path, and a position sensor associated with the element which detects a change in position of the flow-responsive element in response to the fluid flow. The flow-responsive element can be made of a wide variety of materials having the desired properties known to those of ordinary skill in the art.

According to the invention multiple modules are positioned parallel to each other. This allows fluid to be selectively delivered to one of the parallel modules or to another, or to both simultaneously. Moreover, this allows for one of the parallel modules to go undergo recharging while the dialysis machine, dialysis circuit or dialysis flow path is operational. In particular, the parallel modules can have one module inline and the other module offline.

In any embodiment of the invention, the recharging step can be accomplished with one recharger and three valves. In any embodiment of the first, second or third aspects of the invention, the recharging step can be accomplished with one or more recharger and any number of valves. It will be understand that additional combinations of valves and rechargers can be implemented to accomplish any desired online/offline state for any combination of modules or sets of modules in parallel. For example, in any embodiment of the invention, a first set of one or more modules can be in parallel with a second set of one or more modules. As such, the first set of modules can be operated inline and the second set offline. Subsequently, the inline/offline operation can be alternated between the first and second set of parallel modules whereby the first set is offline and the second set is inline. During any offline step, the one or more modules can be recharged. Alternatively, in any embodiment of the invention, the first and second set of modules can both be simultaneously inline and/or offline. Any number of parallel sets of modules containing any number of modules within each set are contemplated by the invention.

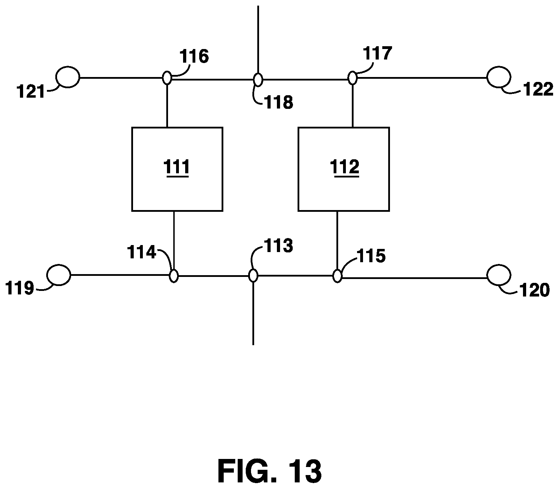

One non-limiting example of the invention is shown in FIG. 13. Fluid can pass to a first valve **113.** The fluid can then be directed either to a second valve **114** or a third valve **115.** The second valve connects to the first module **111.** The third valve connects to the second module **112.** In this way, fluid may be directed into either the first or the second module. The second valve **114** can also connect to a first recharger connector **119.** The third valve **115** can also connect to a second recharger connector **120.** Upon exiting the first module **111,** fluid will pass to fourth valve **116.** Valve **116** can connect to a third recharger connector **121** or a sixth valve **118.** Likewise, upon exiting the second module **112,** fluid passes to fifth valve **117.** Valve **117** can connect to a fourth recharger connector **122,** and the sixth valve **118.** By selectively directing the flow with the valves, fluid can be caused to pass through either of the two modules parallel to each other. Additionally, fluid may be passed between either module and the rechargers.

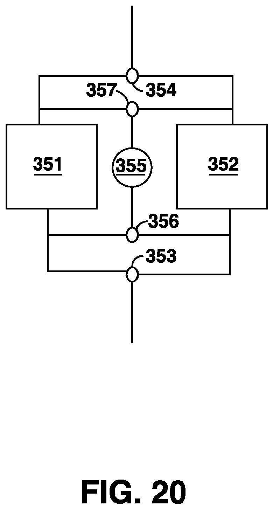

In an alternative embodiment of the invention, shown in FIG. 20, only one recharger is used. Fluid can pass to a first valve **353.** Fluid can then be directed either to the first module **351** or the second module **352.** Upon exiting either the first module **351** or the second module **352,** fluid can pass by the second valve **354.** In this way, fluid can be caused to pass through either the first module **351** or the second module **352.** Additionally, fluid may be circulated between either module and the recharger **355** by utilizing third valve **356** and fourth valve **357** to direct fluid between either module and the recharger **355.** Fluid from the recharger **355** can pass third valve **356,** and can then pass into either the first module **351** or the second module **352.** Fluid exiting either the first module **351** or second module **352** can be directed by fourth valve **357** back to the recharger **355.** Because the modules are parallel, either one may be used or recharged without disrupting the other.

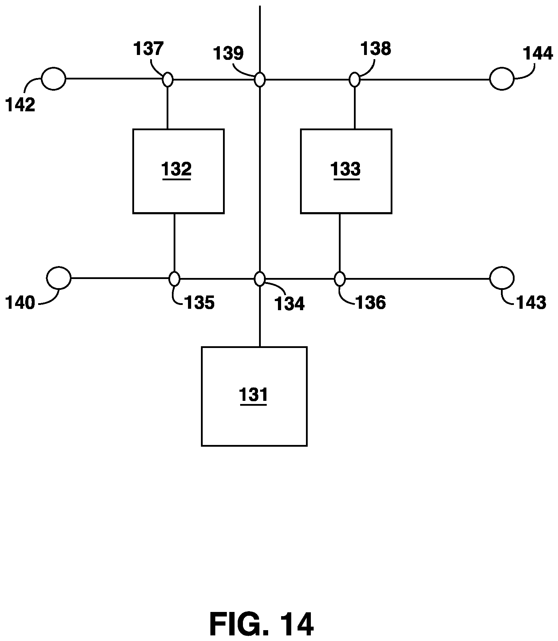

Because it may be advantageous to put different sorbent materials in different modules for easier recharging of the materials, it would be useful to have the parallel modules in series with one or more other modules. In FIG. 14, the first module **131** is in series with the second and third modules, **132** and **133** respectively, which are parallel to each other. Upon leaving the first module **131,** fluid can be directed by the first valve **134** to the second valve **135,** third valve **136,** or sixth valve **139.** If directed to second valve **135,** the fluid can then enter the second module **132.** If directed to third valve **136,** the fluid may then enter the third module 133. If directed toward sixth valve **139,** the fluid will bypass both the second and third modules. Additionally, the second valve **135** and third valve **136** can attach to first recharger connector **140** and second recharger connector **143** respectively. Upon exiting the second module **132,** fluid passes to fourth valve **137,** which is connected to a third recharger connector **142** and sixth valve **139.** Upon exiting the third module **133,** fluid passes to fifth valve **138,** which can connect to a fourth recharger connector **144** and sixth valve **139.** By selectively directing the fluid flow path using the valves, fluid may be caused to pass from the first module through either of the second or third modules or to bypass both the second or third module. The second and third modules are both connected to rechargers, wherein fluid can be passed through either recharger selectively without disrupting the other.

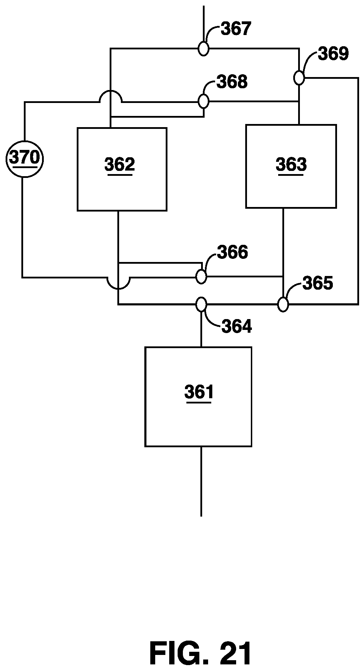

In an alternative embodiment of the invention, a single recharger can be used as shown in FIG. 21. The first module **361** is in series with the second and third modules, **362** and **363** respectively, which are parallel to each other. Fluid leaving the first module **361** can pass valve **364.** The fluid can then be directed towards second module **362,** or second valve **365.** Fluid directed toward the second valve **365** can bypass both the second module **362** and third module **363** through sixth valve **369,** or be directed into third module **363.** In this way, fluid from the first module **361** may be directed through the second module **362,** the third module **363,** or neither module. Additionally, fluid may be circulated between either module and the recharger connector **370** by circulating fluid from the recharger connector **370** to third valve **366** and then through either the second or third module. Fluid from the recharger connector **370** can pass third valve **366,** and then into either the second module **362** or the third module **363.** Because the modules are parallel, either one may be used or recharged without disrupting the other. Fluid exiting the second module **362** or third module **363** can pass through either fourth valve **367** to exit the module, or through fifth valve **368** to circulate with the recharger connector **370.**

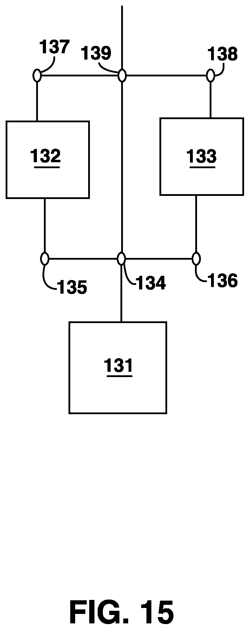

In an alternate embodiment of the invention, a first module **131** can be placed in series with a second module **132** and third module **133,** the latter two of which are parallel to each other, in the absence of a recharger. In FIG. 15, fluid can be directed among and between each of the three modules. This arrangement may be advantageous if different sorbent materials are contained in different modules such that the materials may be recharged without the use of a recharger. Upon leaving the first module **131,** fluid can be directed by the first valve **134** to the second valve **135,** third valve **136,** or sixth valve **139.** If directed to second valve **135,** the fluid can then enter the second module **132.** If directed to third valve **136,** the fluid may then enter the third module **133.** If directed toward sixth valve **139,** the fluid will bypass both the second and third modules.

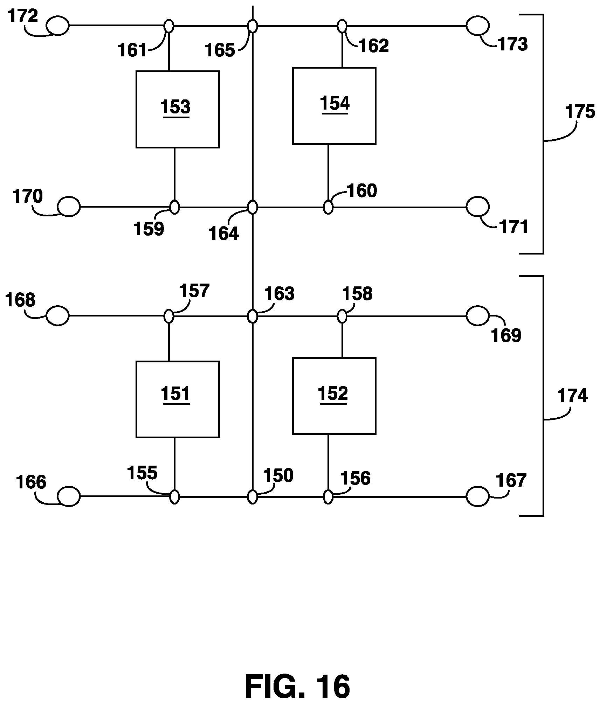

In any embodiment of the invention the sorbent cartridge can be made up of two sets of parallel modules. Each of these two sets of modules can have two modules that are parallel to one another. In FIG. 16, fluid can pass by a first valve **150** before entering the first set of modules **174.** Valve **150** can connect to a second valve **155,** third valve **156** and tenth valve **163.** The second valve **155** can connect to the first module **151,** and also a first recharger connector **166.** The third valve **156** can connect to the second module **152,** and also a second recharger connector **167.** Upon exiting the first module, fluid passes by fourth valve **157,** which can connect to a third recharger connector **168,** and tenth valve **163.** Upon exiting the second module **152,** fluid passes to fifth valve **158,** which can connect to a fourth recharger connector **169** and tenth valve **163.** In this way, fluid can be caused to pass through the first or the second modules of the first set of modules, or to pass through neither of them. Additionally, each module is connected to a recharger, so that each fluid may selectively pass between each module and the proper rechargers.

Similarly, before entering the second set of modules **175,** fluid travels from tenth valve **163** to eleventh valve **164.** Valve **164** can connect to a sixth valve **159,** seventh valve **160** and twelfth valve **165.** The sixth valve **159** can connect to a fifth recharger connector **170** and to the third module **153.** The seventh valve **160** can connect to a sixth recharger connector **171** and fourth module **154.** Upon exiting the third module **153,** fluid passes eighth valve **161,** which can connect to seventh recharger connector **172** and twelfth valve **165.** Upon exiting the fourth module **154,** fluid passes ninth valve **162,** which can connect to an eighth recharger connector **173** and twelfth valve **165.** So fluid may pass through either of the modules in the second set of modules, or it can pass through neither of them. In this way, fluid may selectively travel through either of the modules in the first set of modules, and either of the modules in the second set of modules. Additionally, each module is connected to a recharger so that fluid may selectively pass between the module and the recharger. In any embodiment of the invention, if the parallel pairs **153** and **154,** and **151** and **152** are not identical (i.e., not four of the same module), two recyclers can be configured one for each pair of parallel modules. In any embodiment of the invention, four recyclers can be configured one for each module.

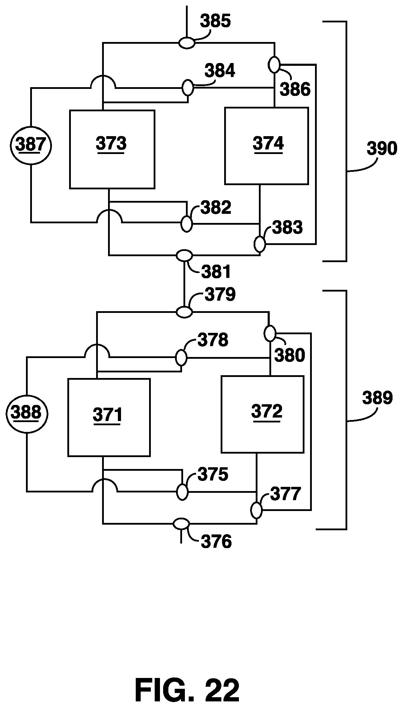

An alternative embodiment of the invention, utilizing a single recharger connector for each set of parallel modules, is shown in FIG. 22. Fluid can pass by a first valve **376** before entering the first set of modules **389.** Valve **376** can connect to a second valve **377,** and first module **371.** The second valve **377** can connect to the second module **372,** or bypass both the first module **371** and second module **372** through sixth valve **380.** Additionally, fluid may be circulated between either module and the recharger connector **388** by circulating fluid from the recharger connector **388** to third valve **375** and then through either the first module **371** or second module **372.** Fluid from the recharger connector **388** can pass third valve **375** and then into the first module **371** or second module **372.** Because the modules are parallel, either one may be recharged or used without disrupting the other. Upon exiting the first module **371** or second module **372,** fluid can pass by fourth valve **379,** to exit the first set of parallel modules, or fluid can pass by fifth valve **378** to circulate with the first recharger connector **388.**

Similarly, before entering the second set of modules **390,** fluid travels to seventh valve **381.** Seventh valve **381** can connect to a third module **373** and eighth valve **383.** The eighth valve **383** can connect to a fourth module **374,** or bypass both the third module **373** and fourth module **374** to twelfth valve **386.** In this way, fluid from the first set of modules **389** can enter the third module **373,** fourth module **374** or bypass both modules. Additionally, fluid may be circulated between either module and the second recharger connector **387** by circulating fluid from second recharger connector **387** to ninth valve **382** and then through either the third module **373** or fourth module **374.** Fluid from the recharger connector **387** can pass ninth valve **382,** and then into either the third module **373** or the fourth module **374.** Because the modules are parallel, either one may be used or recharged without disrupting the other. Fluid exiting the third module **373** or fourth module **374** can pass through either eleventh valve **385** to exit the module, or through tenth valve **384** to circulate with the recharger connector **387.**

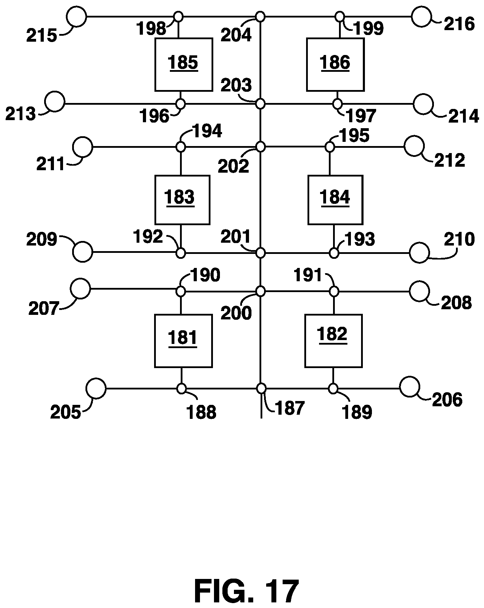

One skilled in the art will recognize that the invention are not limited to systems having two modules in parallel, or two sets of parallel modules in series. Additionally, in any embodiment of the invention, each set of parallel modules may include three or more modules. FIG. 17 shows a sorbent cartridge containing three sets of parallel modules. Fluid passes by a first valve **187** before entering the first set of modules. First valve **187** can connect to a second valve **188,** third valve **189** and fourteenth valve **200.** The second valve **188** can connect to the first module **181,** and also to a first recharger connector **205.** The third valve **189** can connect to the second module **182,** and also a second recharger connector **206.** Upon exiting the first module **181,** fluid passes by fourth valve **190,** which can connect to a third recharger connector **207,** and fourteenth valve **200.** Upon exiting the second module **182,** fluid passes to fifth valve **191,** which can connect to a fourth recharger connector **208** and fourteenth valve **200.** In this way, fluid can be caused to pass through the first or the second modules of the first set of modules, or to pass through neither of them by directing flow directly from the first valve **187** to the fourteenth valve **200.** Additionally, each module is connected to a recharger, so that each fluid may selectively pass between each module and the proper recharger.

Similarly, before entering the second set of modules fluid travels from fourteenth valve **200** to fifteenth valve **201.** Fourteenth valve **200** can connect to a sixth valve **192,** seventh valve **193** and sixteenth valve **202.** The sixth valve **192** can connect to a fifth recharger connector **209** and to the third module **183.** The seventh valve **193** can connect to a sixth recharger connector **210** and fourth module **184.** Upon exiting the third module **183,** fluid passes eighth valve **194,** which can connect to seventh recharger connector **211** and sixteenth valve **202.** Upon exiting the fourth module **184,** fluid passes ninth valve **195,** which can connect to an eighth recharger connector **212** and sixteenth valve **202.** So fluid may pass through either of the modules in the second set of modules, or it can pass through neither of them by directing flow directly from the fifteenth valve **201** to the sixteenth valve **202.**

The sixteenth valve **202** can connect to seventeenth valve **203.** Like the other two sets of modules, before entering the third set, fluid passes by the seventeenth valve **203.** Valve **203** can connect to tenth valve **196,** eleventh valve **197** and eighteenth valve **204.** The tenth valve **196** can connect to the fifth module **185** and a ninth recharger connector **213.** The eleventh valve **197** can connect to the sixth module **186** and tenth recharger connector **214.** Upon exiting the fifth module **185,** fluid passes twelfth valve **198.** Twelfth valve **198** can connect the fifth module **185** with the eleventh recharger connector **215** and eighteenth valve **204.** Upon exiting the sixth module **186,** fluid passes by thirteenth valve **199.** Thirteenth valve **199** can connect the sixth module **186** with the twelfth recharger connector **216** and the eighteenth valve **204.** So fluid can be made to selectively pass through either of the modules in the third set, or bypass both of them by directing flow directly from the seventeenth valve **203** to the eighteenth valve **204.** Additionally, fluid may pass between any of the modules and the rechargers without disrupting the other modules. In certain non-limiting embodiments, if the parallel pairs **181** and **182,** and **183** and **184,** and **185** and **186,** are not identical (i.e., not six of the same module), three recyclers can be configured one for each pair of parallel modules. In other embodiments, six recyclers can be configured one for each module.

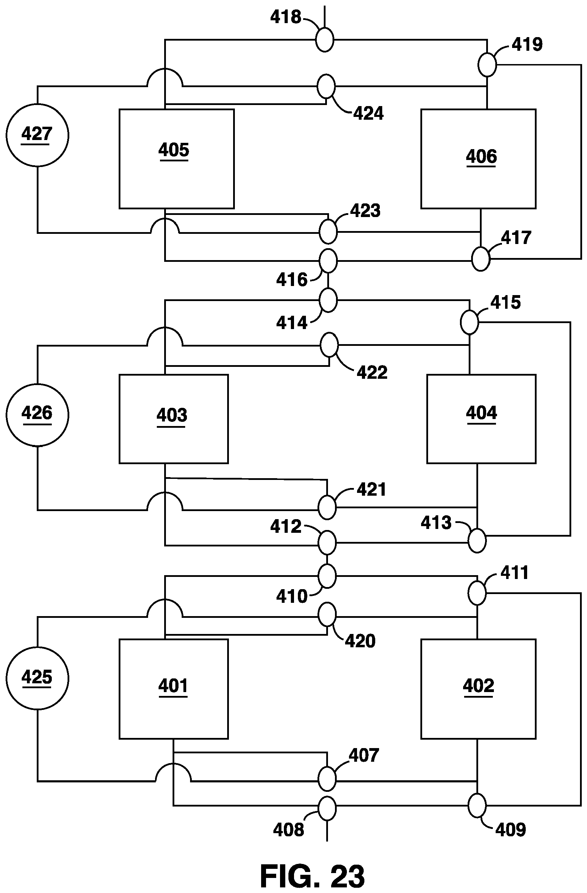

An alternative embodiment of the three module system of the invention, utilizing a single recharger for each set of parallel modules, is shown in FIG. 23. Fluid can pass by a first valve **408** before entering the first set of modules. Valve **408** can connect to a second valve **409,** and first module **401.** The second valve **409** can connect to the second module **402,** or bypass both the first module **401** and second module **402** through sixth valve **411.** Additionally, fluid may be circulated between either module and the recharger connector **425** by circulating fluid from the recharger connector **425** to third valve **407** and then through either the first module **401** or second module **402.** Fluid from the recharger connector **425** can pass third valve **407** and then into the first module **401** or second module **402.** Because the modules are parallel, either one may be recharged or used without disrupting the other. Upon exiting the first module **401** or second module **402,** fluid can pass by fourth valve **410,** to exit the first set of parallel modules, or fluid can pass by fifth valve **420** to circulate with the first recharger connector **425.**

Similarly, before entering the second set of modules fluid travels to seventh valve **412.** Seventh valve **381** can connect to a third module **403** and eighth valve **413.** The eighth valve **413** can connect to a fourth module **404,** or bypass both the third module **403** and fourth module **404** to twelfth valve **415.** In this way, fluid from the first set of modules can enter the third module **403,** fourth module **404** or bypass both modules. Additionally, fluid may be circulated between either module and the second recharger connector **426** by circulating fluid from second recharger connector **426** to ninth valve **421** and then through either the third module **403** or fourth module **404.** Fluid from the recharger connector **426** can pass ninth valve **421,** and then into either the third module **403** or the fourth module **404.** Because the modules are parallel, either one may be used or recharged without disrupting the other. Fluid exiting the third module **403** or fourth module **404** can pass through either eleventh valve **414** to exit the second set of modules, or through tenth valve **422** to circulate with the recharger connector **426.**

Before entering the third set of modules fluid travels to thirteenth valve **416.** Thirteenth valve **416** can connect to a fifth module **405** and fourteenth valve **417.** The fourteenth valve **417** can connect to a sixth module **406,** or bypass both the fifth module **405** and sixth module **406** to eighteenth valve **419.** In this way, fluid from the first set of modules can enter the fifth module **405,** sixth module **406** or bypass both modules. Additionally, fluid may be circulated between either module and the third recharger connector **427** by circulating fluid from third recharger connector **427** to fifteenth valve **423** and then through either the fifth module **405** or sixth module **406.** Fluid from the recharger connector **427** can pass fifteenth valve **423**, and then into either the fifth module **405** or the sixth module **406**. Because the modules are parallel, either one may be used or recharged without disrupting the other. Fluid exiting the fifth module **405** or sixth module **406** can pass through either sixteenth valve **418** to exit the module, or through seventeenth valve **424** to circulate with the recharger connector **427.**

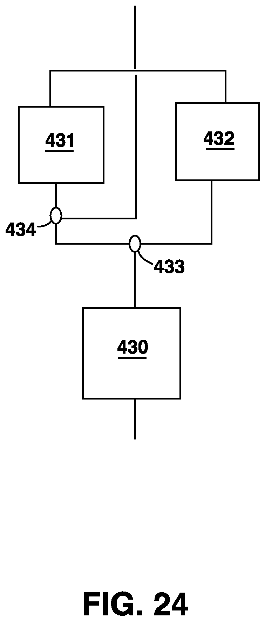

One skilled in the art will understand that the precise number of valves utilized in any embodiment of the invention may be altered without being beyond the scope of the invention. Valves may be added or removed to any of the embodiments of the invention to accomplish the same end. For example, FIG. 24 shows a similar embodiment of the invention as is shown in FIG. 15, but uses only two valves. Fluid leaving the first module **430** passes by the first valve **433.** First valve **433** can direct fluid either to the third module **432,** or to second valve **434.** Second valve **434** can direct fluid either into the second module **431,** or it can cause the fluid to bypass both modules. In this way, fluid from module **430** may be directed into second module **431,** third module **432,** or bypass both modules.

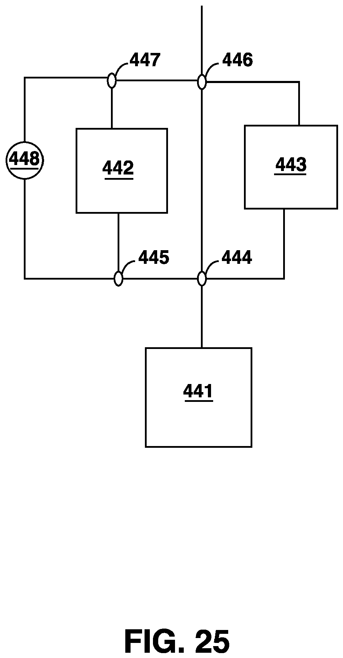

FIG. 25 shows a similar embodiment of the invention as is shown in FIG. 21, but only uses four valves. Fluid leaving the first module **441** passes by first valve **444.** First valve **444** can direct fluid either to the third module **443,** to second valve **445,** or it can cause the fluid to bypass both modules. Second valve **445** can direct fluid either to the recharger connector **448** or into the second module **442.** In this way, fluid from the first module **441** can be directed into the second module **442,** third module **443,** or bypass both modules. Upon exiting the third module **443,** or bypassing both the second module **442** and third module **443,** fluid passes to fourth valve **446,** where it may be directed to another part of the sorbent cartridge. Upon exiting the second module **442,** fluid passes to third valve **447,** where it may be directed to fourth valve **446** and out of the module. Fluid may be circulated between the second module **442** and the recharger connector **448** by utilizing second valve **445** and third valve **447.** Similarly, fluid can be circulated between the recharger connector **448** and the third module **443** by directing fluid by the second valve **445** to the first valve **444,** and on into the third module **443.**

It will be understood that any number of modules can be configured in the invention. For example, in any embodiment of the invention, a sorbent cartridge having four, five, six, seven, or more sets of parallel modules is contemplated.

In any of the embodiments of the invention, the modules may be made either removable or non-removable. Removable modules may be discarded and replaced, recharged out of line or offline from dialysis, or the sorbent material within the module may be discarded, the module refilled with new sorbent material and then reused. This allows for selective discarding or recycling of one or more modules without removing other modules.

For use in sorbent dialysis, the modular sorbent cartridge should be filled with sorbent material. As spent dialysate moves through the cartridge, the sorbent materials selectively remove specific solutes from the dialysate. Various combinations of sorbent material are known in the art to remove toxins from the spent dialysate. For instance, a sorbent cartridge may be filled with layers of activated carbon, hydrous zirconium oxide, alumina, urease, ion exchange resin and zirconium phosphate. The activated carbon removes non-ionic uremic toxins from the dialysate; the hydrous zirconium oxide removes phosphate and fluoride anions; the alumina/urease catalyzes the breakdown of urea into ammonium ions; and the zirconium phosphate removes the ammonium, calcium, potassium and magnesium ions from the spent dialysate. Each of these layers may be recharged after dialysis to return the layer to its original state or usable capacity.

By placing different sorbent materials in different sorbent modules, the individual modules may be recharged or discarded. One skilled in the art will recognize that the precise order of the layers, and which layers go into which module of a modular sorbent cartridge, may be varied without detracting from the invention. For instance, in any embodiment of the invention, the first module may be filled with a layer of activated carbon, a layer of hydrous zirconium oxide, and a layer of alumina/urease, while the second module may be filled with zirconium phosphate. Further, in embodiment of the invention, the sorbent materials may be mixed in the module, as opposed to arranging the material in layers.

Because, for the purpose of recharging the module, calcium and magnesium may be more difficult to remove from the zirconium phosphate, it may be beneficial to remove these ions prior to the dialysate reaching the zirconium phosphate layer. For instance, in any embodiment of the invention, a first module may contain layers of activated carbon, ion exchange resin, hydrous zirconium oxide and alumina/urease, while the second module contains zirconium phosphate. The ion exchange resin will remove the calcium, magnesium and potassium so that the only removable cation that remains in the zirconium phosphate layer is ammonium. If a chelating ion exchange resin is employed, the potassium will pass through the ion exchange resin and be removed by the zirconium phosphate. Potassium should be easier to remove from zirconium phosphate during recharging, and this would allow the use of less of the ion exchange resin.

By placing modules in parallel with one another, one of the parallel modules may be recharged by utilizing the attached rechargers utilizing an alternate duty cycle. For instance, in any embodiment of the invention, if in FIG. 15 the first module **131** contains activated carbon, hydrous zirconium oxide, and alumina/urease and the second module **132** and third module **133** contain zirconium phosphate, then either the second or third module may be selectively recharged in this alternate duty cycle without disrupting the other parallel module.

Similarly, in any embodiment of the invention, the system in FIG. 16 allows for alternate duty recharging of multiple modules. Either of the sub-modules in each module may be recharging without disrupting the other parallel sub-modules.

In any embodiment of the invention, in order to ensure that all of the residual fluid is removed from the reusable modules, valves, bypass lines and wash lines, it may be advantageous to blow a gas, such as argon, air, filtered air, nitrogen, helium, or other gas, through the module. The wash line may be adapted so that a gas may be blown through the module instead of, or in addition to, a wash liquid.

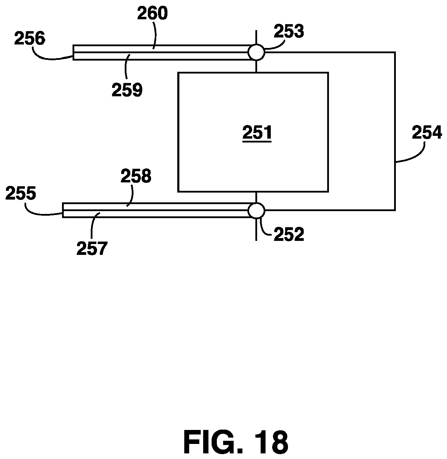

Alternatively, in any embodiment of the invention, a wash line may be divided into two lines as shown in FIG. 18. FIG. 18 shows a first module from an embodiment similar to that shown in FIG. 17 wherein the wash line is adapted to utilize both a liquid and a gas. The first wash line **255** can connect to a first valve **252** positioned before the first module **251.** The first valve **252** can also connect to the first bypass line **254.** The first bypass line **254** can direct either liquid or gas around the first module **251** to the second valve **253.** The first wash line **255** can be further divided into two lines. These lines are the liquid wash line **257,** and the gas wash line **258.** A second wash line **256** can connect to the second valve **253,** and also have both a liquid wash line **259** and a gas wash line **260.** This embodiment of the invention allows both gas and liquid to pass either into the first module, or to bypass the first module.

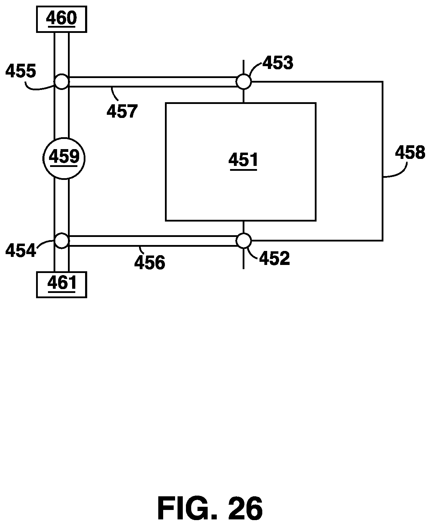

FIG. 26 shows an alternative embodiment of the invention utilizing both a gas and liquid line. The first wash line **456** can connect a first valve **454** to a second valve **452.** The first valve **454** can be connected to the recharger connector **459,** and a fluid collector **461.** The recharger connector can also be attached to third valve **455.** Third valve **455** can connect the gas source **460** and fourth valve **453** via second wash line **457.** Both the second valve **452** and the fourth valve **453** connect to the module **451** and a bypass line **458.** This embodiment of the invention allows both gas and liquid to circulate through the module **451,** or around the module **451.**

In addition to dividing the wash line into a gas wash line and a liquid wash line, in any embodiment of the invention, the wash line may be divided into two different liquid lines. This enables different liquids to travel between the recharger and the modules.

Because the modules in the modular sorbent cartridge may be made detachable, in any embodiment of the invention, it may be beneficial to include an identification component on the detachable module or modules. This identification component may be a bar code, or any other component that will allow for identification of a particular module. The module then can be matched up to a particular patient, cartridge, or other part of the system to eliminate cross contamination.

One skilled in the art will understand that various combinations and/or modifications and variations can be made in the dialysis system depending upon the specific needs for operation under the scope of appended claims.

## Claims

1. A sorbent cartridge, comprising:
at least two modules, wherein the at least two modules are part of a controlled compliant dialysis circuit, at least one module being a reusable module, wherein the at least two modules are positioned parallel to one another, and wherein at least one specified sorbent material is in each of the modules;
the at least two modules are connectable through at least one connector to form the sorbent cartridge; wherein
the modules have one or more connectors selectively fluidly connectable to any one of a fluid flow path, a wash line wherein the wash line is fluidly connectable to a recharger, and a bypass line wherein the bypass line is fluidly connectable to another module or fluid flow path, and
at least one of the modules is configured to be in an offline state by being fluidly connected to one or more recharger, and at least one of the modules is configured to be in an online state by being fluidly connected to any one of the fluid flow path or the bypass line.

2. The sorbent cartridge of claim 1, further comprising valves positioned before and/or after the modules on the connectors to selectively direct flow through the modules, fluid flow paths, wash lines, or bypass lines.

3. The sorbent cartridge of claim 2, wherein a first module is positioned in series before a second and third module, wherein the second and third module are positioned parallel to one another.

4. The sorbent cartridge of claim 2, wherein a first module is positioned in series before a second and third module, wherein the second and third module are positioned parallel to one another; wherein the first module is connected to a first connector; the first connector having a first valve.

5. The sorbent cartridge of claim 2, wherein a first and second module are positioned parallel to one another, a third and fourth module are positioned parallel to one another, and the first and second modules are in series with the third and fourth modules.

6. The sorbent cartridge of claim 2, wherein a first and second module are positioned parallel to one another, a third and fourth module are positioned parallel to one another, a fifth and sixth module are positioned parallel to one another and the first and second modules are in series with the third and fourth modules, and the third and fourth modules are in series with the fifth and sixth modules.

7. The sorbent cartridge of claim 1, wherein the at least one reusable module contains sorbent material selected from the group comprising zirconium phosphate, hydrous zirconium oxide, activated carbon, alumina, urease, and ion exchange resin.

8. The sorbent cartridge of claim 2, wherein the connectors include an access point for a sensor.

## Patentansprüche

1. Sorbenskartusche, die Folgendes umfasst:
wenigstens zwei Module, wobei die wenigstens zwei Module Teil eines gesteuerten konformen Dialysekreislaufs sind und wenigstens ein Modul ein wiederverwendbares Modul ist, wobei die wenigstens zwei Module parallel zueinander positioniert sind und wobei wenigstens ein spezifiziertes Sorbensmaterial in jedem der Module ist;
wobei die wenigstens zwei Module durch wenigstens einen Verbinder verbindbar sind, um die Sorbenskartusche auszubilden; wobei
die Module einen oder mehrere Verbinder aufweisen, die wahlweise fluidisch verbindbar sind mit einem Fluidströmungsweg, einer Waschleitung, wobei die Waschleitung mit einer Anreicherungsvorrichtung fluidisch verbindbar ist, oder einer Umgehungsleitung, wobei die Umgehungsleitung mit einem anderen Modul oder Fluidströmungsweg fluidisch verbindbar ist, und
wenigstens eines der Module konfiguriert ist, um durch eine fluidische Verbindung mit einer oder mehreren Anreicherungsvorrichtungen in einem nicht angeschlossenen Zustand zu sein, und wenigstens eines der Module konfiguriert ist, um durch die fluidische Verbindung mit dem Fluidströmungsweg oder der Umgehungsleitung in einem angeschlossenen Zustand zu sein.

2. Sorbenskartusche nach Anspruch 1, die ferner Ventile umfasst, die vor und/oder nach den Modulen an den Verbindern positioniert sind, um eine Strömung durch die Module, die Fluidströmungswege, die Waschleitungen oder die Umgehungsleitungen wahlweise zu lenken.

3. Sorbenskartusche nach Anspruch 2, wobei ein erstes Modul vor einem zweiten und einem dritten Modul in Reihe positioniert ist, wobei das zweite und das dritte Modul parallel zueinander positioniert sind.

4. Sorbenskartusche nach Anspruch 2, wobei ein erstes Modul vor einem zweiten und einem dritten Modul in Reihe positioniert ist, wobei das zweite und das dritte Modul parallel zueinander positioniert sind; wobei das erste Modul mit einem ersten Verbinder verbunden ist; wobei der erste Verbinder ein erstes Ventil aufweist.

5. Sorbenskartusche nach Anspruch 2, wobei ein erstes und ein zweites Modul parallel zueinander positioniert sind, ein drittes und ein viertes Modul parallel zueinander positioniert sind und das erste und das zweite Modul mit dem dritten und dem vierten Modul in Reihe sind.

6. Sorbenskartusche nach Anspruch 2, wobei ein erstes und ein zweites Modul parallel zueinander positioniert sind, ein drittes und ein viertes Modul parallel zueinander positioniert sind, ein fünftes und ein sechstes Modul parallel zueinander positioniert sind und das erste und das zweite Module mit dem dritten und dem vierten Modul in Reihe sind und das dritte und das vierte Modul mit dem fünften und dem sechsten Modul in Reihe sind.

7. Sorbenskartusche nach Anspruch 1, wobei das wenigstens eine wiederverwendbare Modul ein Sorbensmaterial enthält, das aus der Gruppe ausgewählt ist, die Zirkoniumphosphat, wasserhaltiges Zirkoniumoxid, Aktivkohle, Aluminiumoxid, Urease und lonenaustauschharz umfasst.

8. Sorbenskartusche nach Anspruch 2, wobei die Verbinder einen Zugangspunkt für einen Sensor beinhalten.

## Revendications

1. Cartouche de sorbant, comprenant :
au moins deux modules, les au moins deux modules faisant partie d'un circuit de dialyse conforme régulé, au moins un module étant un module réutilisable, les au moins deux modules étant positionnés parallèlement l'un à l'autre, et au moins un matériau sorbant spécifié se trouvant dans chacun des modules ;
les au moins deux modules peuvent être reliés par l'intermédiaire d'au moins un raccord pour former la cartouche de sorbant ; dans lequel
les modules ont un ou plusieurs raccords pouvant être reliés fluidiquement de manière sélective à l'un quelconque parmi un trajet d'écoulement de fluide, une ligne de lavage dans laquelle la ligne de lavage peut être reliée fluidiquement à un chargeur, et une conduite de dérivation dans laquelle la conduite de dérivation peut être reliée fluidiquement à un autre module ou à un trajet d'écoulement de fluide, et
au moins un des modules est conçu pour être dans un état hors ligne en étant relié fluidiquement à un ou plusieurs chargeurs, et au moins un des modules est conçu pour être dans un état en ligne en étant relié fluidiquement à l'un quelconque des trajets d'écoulement de fluide ou de la ligne de dérivation.

2. Cartouche de sorbant selon la revendication 1, comprenant en outre des soupapes positionnées avant et/ou après les modules sur les raccords pour diriger sélectivement l'écoulement à travers les modules, les trajets d'écoulement de fluide, les lignes de lavage ou les lignes de dérivation.

3. Cartouche de sorbant selon la revendication 2, dans laquelle un premier module est positionné en série avant un deuxième et un troisième module, les deuxième et troisième modules étant positionnés parallèlement l'un à l'autre.

4. Cartouche de sorbant selon la revendication 2, dans laquelle un premier module est positionné en série avant un deuxième et un troisième module, les deuxième et troisième modules étant positionnés parallèlement l'un à l'autre ; le premier module est relié à un premier raccord ; le premier raccord ayant une première soupape.

5. Cartouche de sorbant selon la revendication 2, dans laquelle un premier et un deuxième module sont positionnés parallèlement l'un à l'autre, un troisième et un quatrième module sont positionnés parallèlement l'un à l'autre, et les premier et deuxième modules sont en série avec les troisième et quatrième modules.

6. Cartouche de sorbant selon la revendication 2, dans laquelle un premier et un deuxième module sont positionnés parallèlement l'un à l'autre, un troisième et un quatrième module sont positionnés parallèlement l'un à l'autre, un cinquième et un sixième module sont positionnés parallèlement l'un à l'autre et les premier et deuxième modules sont en série avec les troisième et quatrième modules, et les troisième et quatrième modules sont en série avec les cinquième et sixième modules.

7. Cartouche de sorbant selon la revendication 1, dans laquelle l'au moins un module réutilisable contient un matériau sorbant choisi dans le groupe comprenant le phosphate de zirconium, l'oxyde de zirconium hydraté, le charbon actif, l'alumine, l'uréase et la résine échangeuse d'ions.

8. Cartouche de sorbant selon la revendication 2, dans laquelle les raccords comportent un point d'accès pour un capteur.
